# EUROPÄISCHE PATENTSCHRIFT

(11) **EP 2 238 148 B1**
(45) Veröffentlichungstag und Bekanntmachung des Hinweises auf die Patenterteilung: **24.10.2012**
(21) Anmeldenummer: 08866797.7
(22) Anmeldetag: 23.12.2008
(51) Int. Cl.: C07J 53/00, A61K 31/58, A61P 5/34, A61P 5/26, A61P 5/42

(54) **15,16-METHYLEN-17-(1'-PROPENYL)-17,3'-OXIDOESTRA-4-EN-3-ON-DERIVAT, DESSEN VERWENDUNG UND DAS DERIVAT ENTHALTENDE ARZNEIMITTEL**
15,16-METHYLENE-17-(1'-PROPENYL)-17,3'-OXIDOESTRA-4-EN-3-ONE DERIVATIVE, USE THEREOF, AND MEDICAMENT CONTAINING SAID DERIVATIVE
DÉRIVÉ DE 15,16-MÉTHYLÈNE-17-(1'-PROPÉNYL)-17,3'-OXYDOESTRA-4-ÈNE-3-ONE, SON UTILISATION ET MÉDICAMENTS CONTENANT CE DÉRIVÉ

(30) Priorität: 29.12.2007 DE 102007063496
(43) Veröffentlichungstag der Anmeldung: 13.10.2010
(73) Patentinhaber: Bayer Intellectual Property GmbH, 40789 Monheim (DE)
(72) Erfinder: KLAR, Ulrich, 13503 Berlin (DE); KUHNKE, Joachim, 14482 Potsdam (DE); BOHLMANN, Rolf, 14055 Berlin (DE); HÜBNER, Jan, 10317 Berlin (DE); RING, Sven, 07749 Jena (DE); FRENZEL, Thomas, 65719 Hofheim (DE); MENGES, Frederik, 69198 Schriesheim (DE); BORDEN, Steffen, 13355 Berlin (DE); MUHN, Hans, Peter, 13465 Berlin (DE); PRELLE, Katja, 13465 Berlin (DE)
(74) Vertreter: Bayer Intellectual Property GmbH
(86) Internationale Anmeldenummer: PCT/EP2008/011164
(87) Internationale Veröffentlichungsnummer: WO 2009/083271

(56) Entgegenhaltungen:
- EP-A- 0 245 170
- EP-A- 0 277 089
- WO-A-2006/072467
- DE-A1-102006 030 416
- NICKISCH K ET AL: "Aldosterone antagonists. 4. Synthesis and activities of steroidal 6,6-ethylene-15,16-methylene 17-spirolactones" JOURNAL OF MEDICINAL CHEMISTRY, AMERICAN CHEMICAL SOCIETY, WASHINGTON, US, Bd. 34, 1. Januar 1991 (1991-01-01), Seiten 2464-2468, XP002375413 ISSN: 0022-2623

## Beschreibung

Die Erfindung betrifft neue 15,16-Methyten-17-(1'-propenyl)-17-3'-oxidoestra-4-en-3-on-Derivate mit gestagener Wirkung, deren Verwendung sowie die Derivate enthaltende Arzneimittel, beispielsweise zur Behandlung von prä-, peri- und postmenopausalen sowie von prämenstruellen Beschwerden.

Aus der Literatur sind Verbindungen mit gestagener, antimineralcorticoider, antiandrogener oder antiestrogener Wirkung auf Basis eines Steroidgerüstes bekannt, welche beispielsweise von 19-Nor-androst-4-en-3-on oder einem Derivat davon abgeleitet sind (die Nummerierung des Steroidgerüstes ist beispielsweise Fresenius/Görlitzer 3.Aufl. 1991 "Organisch-chemische Nomenklatur" S. 60 ff. zu entnehmen).

So offenbart WO 2006/072467 A1 die als Gestagen wirkende Verbindung 6β,7β-15β,16β-Dimethylen-3-oxo-17-pregn-4-en-21,17β-carbolacton (Drospirenon), welche beispielsweise in einem oralen Kontrazeptivum sowie einem Präparat zur Behandlung postmenopausaler Beschwerden verwendet wurde. Aufgrund seiner vergleichsweise geringen Affinität zum Gestagenrezeptor und seiner vergleichsweise hohen Ovulationshemmdosis ist Drospirenon in dem Kontrazeptivum jedoch in der relativ hohen täglichen Dosis von 3 mg enthalten. Drospirenon zeichnet sich darüber hinaus auch dadurch aus, dass es zusätzlich zur gestagenen Wirkung über aldosteronantagonistische (antimineralcorticoide) sowie antiandrogene Wirkung verfügt. Diese beiden Eigenschaften machen Drospirenon in seinem pharmakologischen Profil dem natürlichen Gestagen Progesteron sehr ähnlich, welches aber anders als Drospirenon nicht ausreichend oral bioverfügbar ist. Um die zu verabreichende Dosis zu senken, werden in WO 2006/072467 A1 weiter ein 18-Methyl-19-nor-17-pregn-4-en-21,17-carbolacton sowie diese enthaltende pharmazeutische Präparate vorgeschlagen, welche über eine höhere gestagene Potenz als Drospirenon verfügen.

Daneben offenbart beispielsweise US-A 3,705,179 Steroide, welche eine antiandrogene Aktivität aufweisen und sich zur Behandlung von Krankheiten eignen, die im Zusammenhang mit Androgenen stehen.

In EP 0 245 170 A1 sind ferner Steroidverbindungen offenbart, in denen in 17-Stellung ein ungesättigter Spiroether und in 11-Stellung ein aromatischer Rest enthalten sind. Die Wirkung dieser Verbindungen wird als progestomimetisch oder antiprogestomimetisch, androgen oder antiandrogen sowie antiglucocorticoid angegeben.

Die Aufgabe der vorliegenden Erfindung ist es, Verbindungen zur Verfügung zu stellen, die über eine starke Bindung an den Gestagenrezeptor verfügen. Außerdem sollen die Verbindungen bevorzugt auch eine antimineralcorticoide Wirkung sowie eine im Hinblick auf den Androgenrezeptor neutrale bis leicht androgene Wirkung aufweisen. Ein weiteres wesentliches Ziel der vorliegenden Erfindung besteht auch darin, ein ausgewogenes Wirkungsprofil hinsichtlich der gestagenen Wirkung zur antimineralcorticoiden Wirkung dergestalt zu erreichen, dass das Verhältnis der gestagenen zur antimineralcorticoiden Wirkung geringer ist als bei Drospirenon.

Diese Aufgabe wird durch die erfindungsgemäßen 15,16-Methylen-17-(1'-propenyl)-17-3'-oxidoestra-4-en-3-on-Derivate gemäß Anspruch 1, die Verwendung der erfindungsgemäßen Derivate gemäß Anspruch 12 sowie ein mindestens ein erfindungsgemäßes Derivat enthaltendes Arzneimittel gemäß Anspruch 14, insbesondere zur oralen Kontrazeption und zur Behandlung von prä-, peri- und postmenopausalen Beschwerden, gelöst. Vorteilhafte Ausführungsformen der Erfindung sind in den Unteransprüchen angegeben.

Die Nummerierung des C-Gerüstes der erfindungsgemäßen Derivate mit den allgemeinen chemischen Formel I folgt in üblicher Weise der Nummerierung eines Steroidgerüstes, beispielsweise beschrieben in Fresenius, a.a.O. Die Nummerierung der in den Ansprüchen angegebenen Reste entspricht in analoger Weise ihrer Bindungsposition am C-Gerüst der Derivate, soweit dies R⁴, R⁶, R⁷ und R¹⁸ betrifft. So bindet beispielsweise der Rest R⁴ an die C⁴-Position des erfindungsgemäßen Derivats.

Hinsichtlich der zu Z definierten Gruppen binden die Gruppen NOR' und NNHSO₂R' jeweils mit einer Doppelbindung über N an das C-Gerüst des Derivats gemäß =NOR' bzw. =NNH-SO₂R'. OR' in NOR' und NHSO₂R' in NNHSO₂R' können syn- oder antiständig stehen.

Unter Alkyl in R', R^{6a}, R6b, R⁷, R¹⁸, R¹⁹, R²⁰, R^{21a}, R^{21b} und R²² sowie in anderen Fällen sind gerad- oder verzweigtkettige Alkylgruppen mit der angegebenen Anzahl an Kohlenstoffatomen oder gegebenenfalls 1-10 Kohlenstoffatomen zu verstehen, wie beispielsweise Methyl, Ethyl, Propyl, Isopropyl, Butyl, Isobutyl, tert.-Butyl, Pentyl, Isopentyl, Neopentyl, Hexyl, Heptyl, Decyl. Unter Alkyl in R¹⁸ ist insbesondere Methyl, Ethyl, Propyl oder 1-sopropyl und unter R²² Methyl, Ethyl, Propyl, Isopropyl, Butyl, Isobutyl, tert.-Butyl, Pentyl, Isopentyl, Neopentyl, Hexyl, zu verstehen.

Unter Alkenyl in R^{6a}, R^{6b} und R⁷ sind gerad- oder verzweigtkettige Alkenylgruppen mit 2-10 Kohlenstoffatomen zu verstehen, wie beispielsweise Vinyl, Propenyl, Butenyl, Pentenyl, Isobutenyl, Isopentenyl.

Unter Alkinyl in R^{6a}, R^{6b} und R⁷ sind gerad- oder verzweigtkettige Alkinylgruppen mit 2-10 Kohlenstoffatomen zu verstehen, wie beispielsweise Ethinyl, Propinyl, Butinyl, Pentinyl, Isobutinyl, Isopentinyl.

Unter Cycloalkyl in R⁷ sind Cycloalkylgruppen mit 3-6 Kohlenstoffatomen zu verstehen, beispielsweise Cyclopropyl, Cyclobutyl, Cyclopentyl und Cyclohexyl.

Als Aralkylreste kommen beispielsweise Benzyl, Phenylethyl, Naphthylmethyl, Naphthylethyl, Furylmethyl, Thienylethyl, Pyridylpropyl in Betracht.

Soweit Alkoxy (O-Alkyl) erwähnt wird, handelt es sich um Alkoxygruppen mit 1-4 Kohlenstoffatomen. Alkoxy kann insbesondere Methoxy, Ethoxy und Propoxy sein.

Soweit Acyl (CO-Alkyl) erwähnt wird, handelt es sich um Acylgruppen mit 1-20 Kohlenstoffatomen. Acyl kann insbesondere Formyl, Acetyl, Propionyl und Butyryl sein.

Soweit Acyloxy (O-CO-Alkyl) erwähnt wird, handelt es sich um Acyloxygruppen mit 1-20 Kohlenstoffatomen. Acyloxy kann insbesondere Formyloxy, Acetyloxy, Propionyloxy und Butyryloxy sein.

Halogen bedeutet Fluor, Chlor oder Brom. Unter diesen ist Chlor bevorzugt.

Die vorliegende Erfindung betrifft 15,16-Methylen-17-(1'-propenyl)-17-3'-oxidoestra-4-en-3-on-Derivate mit der allgemeinen chemischen Formel I: 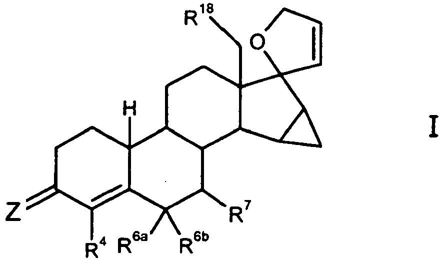 worin
- Z: ausgewählt ist aus der Gruppe, umfassend Sauerstoff, zwei Wasserstoffatome, NOR' und NNHSO₂R', worin R' Wasserstoff, C₁-C₁₀Alkyl, Aryl oder C₇-C₂₀-Aralkyl ist,
- R⁴: ausgewählt ist aus der Gruppe, umfassend Wasserstoff" Fluor, Chlor oder Brom ferner entweder:
- R^{6a}, R^{6b}: jeweils unabhängig voneinander ausgewählt sind aus der Gruppe, umfassend Wasserstoff, C₁-C₁₀-Alkyl, C₂-C₁₀-Alkenyl und C₂-C₁₀-Alkinyl, oder gemeinsam Methylen oder 1,2-Ethandiyl bilden und
- R⁷: ausgewählt ist aus der Gruppe, umfassend Wasserstoff, C₁-C₁₀-Alkyl, C₃-C₆-Cycloalkyl, C₂-C₁₀-Alkenyl und C₂-C₁₀-Alkinyl,
- oder: R^{6a}, R⁷: gemeinsam Sauerstoff oder eine Methylengruppe bilden oder unter Bildung einer Doppelbindung zwischen C⁶ und C⁷ entfallen und
- R^{6b}: ausgewählt ist aus der Gruppe, umfassend Wasserstoff, C₁-C₁₀-Alkyl, C₂-C₁₀-Alkenyl und C₂-C₁₀-Alkinyl,
- R¹⁸: ausgewählt ist aus der Gruppe, umfassend Wasserstoff und C₁-C₃-Alkyl.

Ferner betrifft die Erfindung auch die Solvate, Hydrate und Salze der erfindungsgemäße Derivate, einschließlich aller Kristallmodifikationen und aller Stereoisomere dieser Derivate.

Gemäß einer bevorzugten Ausführungsform der Erfindung ist Z ausgewählt aus der Gruppe, umfassend Sauerstoff, NOR' und NNHSO₂R'.

Gemäß einer weiter bevorzugten Ausführungsform der Erfindung steht Z für Sauerstoff.

Gemäß einer weiter bevorzugten Ausführungsform der Erfindung ist R⁴ ausgewählt aus der Gruppe, umfassend Wasserstoff und Chlor.

Gemäß einer weiter bevorzugten Ausführungsform der Erfindung bilden R^{6a} und R^{6b} gemeinsam 1,2-Ethandiyl oder sind jeweils Wasserstoff.

Gemäß einer weiter bevorzugten Ausführungsform der Erfindung ist R⁷ ausgewählt aus der Gruppe, umfassend Wasserstoff, Methyl, Ethyl und Vinyl.

Gemäß einer weiter bevorzugten Ausführungsform der Erfindung bilden R^{6a} und R⁷ gemeinsam eine Methylengruppe.

Gemäß einer weiter bevorzugten Ausführungsform der Erfindung entfallen R^{6a} und R⁷ unter Bildung einer Doppelbindung zwischen C⁶ und C⁷.

Gemäß einer weiter bevorzugten Ausführungsform der Erfindung ist R¹⁸ ausgewählt aus der Gruppe, umfassend Wasserstoff und Methyl.

Besonders bevorzugt sind Verbindungen mit der allgemeinen chemischen Formel I, worin:
- Z: Sauerstoff oder NOR' ist, wobei R' Wasserstoff, C₁-C₆-Alkyl, Aryl oder C₇-C₁₂-Aralkyl ist,
R⁴ Wasserstoff Fluor, Chlor oder Brom ist,
ferner entweder:
- R^{6a}, R^{6b}: jeweils unabhängig voneinander ausgewählt sind aus der Gruppe, umfassend Wasserstoff, C₁-C₆-Alkyl, C₂-C₆-Alkenyl, C₂-C₆-Alkinyl, oder gemeinsam Methylen oder 1,2-Ethandiyl bilden und
- R⁷: Wasserstoff, C₁-C₆-Alkyl, C₃-C₆-Cycloalkyl, C₂-C₆-Alkenyl oder C₂-C₆-Alkinyl ist,
oder:
- R^{6a}, R⁷: gemeinsam eine Methylengruppe bilden oder unter Bildung einer Doppelbindung zwischen C⁶ und C⁷ entfallen und
- R^{6b}: Wasserstoff, C₁-C₆-Alkyl, C₂-C₆-Alkenyl oder C₂-C₆-Alkinyl ist und
- R¹⁸: Wasserstoff oder C₁-C₂-Alkyl ist,
wobei auch in diesem Falle die Solvate, Hydrate und Salze der erfindungsgemäße Derivate, einschließlich aller Kristallmodifikationen und aller Stereoisomere dieser Derivate einbezogen sind.
Weiter besonders bevorzugt sind Verbindungen mit der allgemeinen chemischen Formel I, worin
- Z: Sauerstoff oder NOR' ist, wobei R' Wasserstoff oder C₁-C₃-Alkyl ist,
- R⁴: Wasserstoff, Chlor oder Brom ist,
ferner entweder:
- R^{6a}, R^{6b}: jeweils unabhängig voneinander ausgewählt sind aus der Gruppe, umfassend Wasserstoff, C₁-C₃-Alkyl und C₂-C₄-Alkenyl, oder gemeinsam Methylen oder 1,2-Ethandiyl bilden und
- R⁷: Wasserstoff, C₁-C₄-Alkyl, C₃-C₄-Cycloalkyl oder C₂-C₄-Alkenyl ist,
oder:
- R^{6a}, R⁷: gemeinsam eine Methylengruppe bilden oder unter Bildung einer Doppelbindung zwischen C⁶ und C⁷ entfallen und
- R^{6b}: Wasserstoff, C₁-C₃-Alkyl oder C₂-C₄-Alkenyl ist und
- R¹⁸: Wasserstoff oder Methyl ist,
wobei auch in diesem Falle die Solvate, Hydrate und Salze der erfindungsgemäße Derivate, einschließlich aller Kristallmodifikationen und aller Stereoisomere dieser Derivate einbezogen sind.

Hiermit werden ausdrücklich alle möglichen Stereoisomere sowie Isomerengemische, einschließlich Racemate, der Verbindungen mit der allgemeinen chemischen Formel I ausdrücklich mit einbezogen. Jeder der genannten Substituenten am Steroid-Grundgerüst kann sowohl in einer α- als auch in einer β-Stellung vorliegen. Außerdem können auch die Substituenten am Steroid-Grundgerüst, die eine Doppelbindung enthalten und in denen die Doppelbindung an jedem Kohlenstoffatom mindestens einen Substituenten, der nicht Wasserstoff ist, trägt, sowohl E- als auch Z-konfiguriert vorliegen. An zwei benachbarte Kohlenstoffatome des Gerüstes gebundene Gruppen, beispielsweise ein Sauerstoffatom, Methylen oder 1,2-Ethandiyl werden entweder in α,α-Stellung oder in β,β-Stellung gebunden.

Ausdrücklich mit einbezogen sind auch alle Kristallmodifikationen der Verbindung mit der allgemeinen chemischen Formel I.

Ausdrücklich mit einbezogen sind auch erfindungsgemäße Derivate in Form von Solvaten, insbesondere von Hydraten, wobei die erfindungsgemäßen Verbindungen demgemäß polare Lösungsmittel, insbesondere von Wasser, als Strukturelement des Kristallgitters der erfindungsgemäßen Verbindungen enthalten. Das polare Lösungsmittel, insbesondere Wasser, kann in einem stöchiometrischen oder auch unstöchiometrischen Verhältnis vorliegen. Bei stöchiometrischen Solvaten, Hydraten spricht man auch von Hemi-, (Semi-), Mono-, Sesqui-, Di-, Tri-, Tetra-, Penta-, usw. Solvaten oder Hydraten.

Ist eine saure Funktion enthalten, sind als Salze die physiologisch verträglichen Salze organischer und anorganischer Basen geeignet, wie beispielsweise die gut löslichen Alkali- und Erdalkalisalze, sowie die Salze von N-Methyl-glukamin, D-Methyl-glukamin, Ethylglutamin, Lysin, 1,6-Hexadiamin, Ethanolamin, Glukosamin, Sarkosin, Serinol, Trishydroxy-methyl-aminomethan, Aminopropandiol, Sovak-Base, 1-Amino-2,3,4-butantriol. Ist eine basische Funktion enthalten, sind die physiologisch verträglichen Salze organischer und anorganischer Säuren geeignet, wie von Salzsäure, Schwefelsäure, Phosphorsäure, Citronensäure, Weinsäure u.a.

Es wurde gefunden, dass die erfindungsgemäßen Verbindungen bzw. Derivate eine gute gestagene Wirkung aufweisen. Außerdem interagieren einige interessante erfindungsgemäße Verbindungen mit dem Mineralcorticoidrezeptor und sind in der Lage, eine antagonistische Wirkung zu vermitteln. Ferner weisen die erfindungsgemäßen Verbindungen im Hinblick auf den Androgenrezeptor eine neutrale bis leicht androgene Wirkung auf. Eine weitere Eigenschaft der überwiegenden Anzahl der Verbindungen besteht darin, dass die Bindungen dieser Verbindungen an den Progesteronrezeptor und an den Mineralcorticoidrezeptor relativ zueinander ausgewogen sind, und zwar dergestalt, dass bei ihnen das Verhältnis der Bindungsfähigkeit zum Progesteronrezeptor zur Bindungsfähigkeit zum Mineralcorticoidrezeptor geringer ist als bei Drospirenon. Somit ist die antimineralcorticoide Wirkung dieser Verbindungen bei gegebener gestagener Wirkung geringer als bei Drospirenon. Wird die Dosierung einer gegebenen erfindungsgemäßen Verbindung aufgrund von deren gestagener Wirkung festgelegt, so ist die antimineralcorticoide Wirkung dieser Verbindung bei dieser Dosierung somit geringer als bei Drospirenon.

Die nachstehend genannten Verbindungen sind insbesondere bevorzugt (zusätzlich ist eine Verweisung auf die weiter unten beschriebenen Synthesebeispiele aufgenommen):

| | |
|---|---|
| 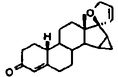 | 17α-(1'-Propenyl)-15α,16α-methylen-17β-3'-oxidoestra-4-en-3-on (Beispiel 12) |
| | 17α-(1'-Propenyl)-15β,16β-methylen-17β-3'-oxidoestra-4-en-3-on (Beispiel 8) |
| 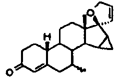 | 7α-Methyl-15α,16α-methylen-17α-(1'-propenyl)-17β-3'-oxidoestra-4-en-3-on (Beispiel 14) |
| | 7α-Methyl-15β,16β-methylen-17α-(1'-propenyl)-17β-3'-oxidoestra-4-en-3-on |
| | 7β-Methyl-15α,16α-methylen-17α-(1'-propenyl)-17α-3'-oxidoestra-4-en-3-on |
| | 7β-Methyl-15β,16β-methylen-17α-(1'-propenyl)-17β-3'-oxidoestra-4-en-3-on |
| 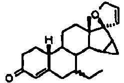 | 7α-Ethyl-15α,16α-methylen-17α-(1'-propenyl)-17β-3'-oxidoestra-4-en-3-on (Beispiel 15A) |
| | 7α-Ethyl-15β,16β-methylen-17a-(1'-propenyl)-17β-3'-oxidoestra-4-en-3-on |
| | 7β-Ethyl-15α,16α-methylen-17α-(1'-propenyl)-17β-3'-oxidoestra-4-en-3-on (Beispiel 15B) |
| | 7β-Ethyl-15B,16β-methylen-17α-(1'-propenyl)-17β-3'-oxidoestra-4-en-3-on |
| 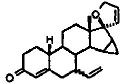 | 7α-Vinyl-15α,16α-methylen-17α-(1'-propenyl)-17β-3'-oxidoestra-4-en-3-on (Beispiel 16A) |
| | 7α-Vinyl-15β,16β-methylen-17α-(1'-propenyl)-17β-3'-oxidoestra-4-en-3-on |
| | 7β-Vinyl-15a,16α-methylen-17α-(1'-propenyl)-17β-3'-oxidoestra-4-en-3-on (Beispiel 16B) |
| | 7β-Vinyl-15β,16β-methylen-17α-(1'-propenyl)-17β-3'-oxidoestra-4-en-3-on |
| 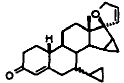 | 7α-Cyclopropyl-15α,16α-methylen-17α-(1'-propenyl)-17β-3'-oxidoestra-4-en-3-on (Beispiel 17A) |
| | 7α-Cyclopropyl-15β,16β-methylen-17a-(1'-propenyl)-17β-3'-oxidoestra-4-en-3-on |
| | 7β-Cyclopropyl-15α,16α-methylen-17a-(1'-propenyl)-17β-3'-oxidoestra-4-en-3-on (Beispiel 17B) |
| | 7β-Cyclopropyl-15β,16β-methylen-17β-(1'-propenyl)-17β-3'-oxidoestra-4-en-3-on |
| 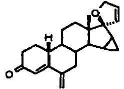 | 6-Methylen-15α,16α-methylen-17α-(1'-propenyl)-17β-3'-oxidoestra-4-en-3-on |
| | 6-Methylen-15β,16β-methylen-17α-(1'-propeny)-17β-3'-oxidoestra-4-en-3-on |
| 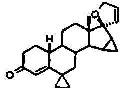 | 6,6-(1,2-Ethandiyl)-15α,16α-methylen-17α-(1'-propenyl)-17β-3'-oxidoestra-4-en-3-on |
| | 6,6-(1,2-Ethandiyl)-15β,16β-methylen-17α-(1'-propenyl)-17β-3'-oxidoestra-4-en-3-on |
| 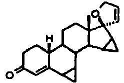 | 6α,7α;15α,16α-Bismethylen-17α-(1'-propenyl)-17β-3'-oxidoestra-4-en-3-on |
| | 6β,7β;15α,17α-Bismethylen-17α-(1'-propenyl)-17β-3'-oxidoestra-4-en-3-on |
| | 6α,7α;15β,16β-Bismethylen-17α-(1'-propenyl)-17β-3'-oxidoestra-4-en-3-on |
| | 6β,7β;15β,16β-Bismethylen-17α-(1'-propenyl)-17β-3'-oxidoestra-4-en-3-on |
| 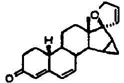 | 17α-(1'-Propenyl)-15α, 16α-methylen-17β-3'-oxidoestra-4,6-dien-3-on (Beispiel 13) |
| | 17α-(1'-Propenyl)-15β,16β-methylen-17β-3'-oxidoestra-4,6-dien-3-on (Beispiel 11) |
| | |
| 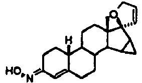 | (E/Z)-3-(Hydroxyimino)-17α-(1'-propenyl)-15α,16α-methylen-17β-3'-oxidoestra-4-en |
| | (E/Z)-3-(Hydroxyimino)-17α-(1'-propenyl)-15β,16β-methylen-17β-3'-oxidoestra-4-en |
| 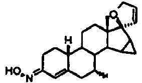 | (E/Z)-3-(Hydroxyimino)-7α-methyl-15α,16α-methylen-17α-(1'-propenyl)-17β-3'-oxidoestra-4-en |
| | (E/Z)-3-(Hydroxyimino)-7α-methyl-15β,16β-methylen-17a-(1'-propenyl)-17β-3'-oxidoestra-4-en |
| | (E/Z)-3-(Hydroxyimino)-7β-methyl-15α,16α-methylen-17a-(1'-propenyl)-17β-3'-oxidoestra-4-en |
| | (E/Z)-3-(Hydroxyimino)-7β-methyl-15β,16β-methylen-17α-(1'-propenyl)-17β-3'-oxidoestra-4-en |
| 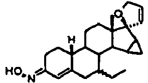 | (E/Z)-3-(Hydroxyimino)-7α-ethyl-15α,16α-methylen-17α-(1'-propenyl)-17β-3'-oxidoestra-4-en |
| | (E/Z)-3-(Hydroxyimino)-7α-ethyl-15β,16β-methylen-17α-(1'-propenyl)-17β-3'-oxidoestra-4-en |
| | (E/Z)-3-(Hydroxyimino)-7β-ethyl-15α,16α-methylen-17α-(1'-propenyl)-17β-3'-oxidoestra-4-en |
| | (E/Z)-3-(Hydroxyimino)-7β-ethyl-15β,16β-methylen-17α-(1'-propenyl)-17β-3'-oxidoestra-4-en |
| | (E/Z)-3-(Hydroxyimino)-7a-vinyl-15α,16α-methylen-17α-(1'-propenyl)-17β-3'-oxidoestra-4-en |
| | (E/Z)-3-(Hydroxyimino)-7a-vinyl-15β,16β-methylen-17α-(1'-propenyl)-17β-3'-oxidoestra-4-en |
| | (E/Z)-3-(Hydroxyimino)-7β-vinyl-15α,16α-methylen-17α-(1'-propenyl)-17β-3'-oxidoestra-4-en |
| | (E/Z)-3-(Hydroxyimino)-7β-vinyl-15β,16β-methylen-17α-(1'-propenyl)-17β-3'-oxidoestra-4-en |
| 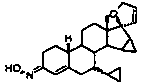 | (E/Z)-3-(Hydroxyimino)-7α-cyclopropyl-15α,16α-methylen-17α-(1'-propenyl)-17β-3'-oxidoestra-4-en |
| | (E/Z)-3-(Hydroxyimino)-7α-cyclopropyl-15β,16β-methylen-17α-(1'-propenyl)-17β-3'-oxidoestra-4-en |
| | (E/Z)-3-(Hydroxyimino)-7β-cyclopropyl-15α,16α-methylen-17α-(1'-propenyl)-17β-3'-oxidoestra-4-en |
| | (E/Z)-3-(Hydroxyimino)-7β-cyclopropyl-15β,16β-methylen-17α-(1'-propenyl)-17β-3'-oxidoestra-4-en |
| 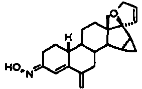 | (E/Z)-3-(Hydroxyimino)-6-methylen-17α-(1'-propenyl)-15α,16α-methylen-17β-3'-oxidoestra-4-en |
| | (E/Z)-3-(Hydroxyimino)-6-methylen-17α-(1'-propenyl)-15β,16β-methylen-17β-3'-oxidoestra-4-en |
| 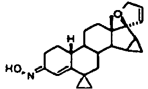 | (E/Z)-3-(Hydroxyimino)-6,6-(1,2-ethandiyl)-17α-(1'-propenyl)-15α,16α-methylen-17β-3'-oxidoestra-4-en |
| | (E/Z)-3-(Hydroxyimino)-6,6-(1,2-ethandiyl)-17a-(1'-propenyl)-15β,16β-methylen-17β-3'-oxidoestra-4-en |
| 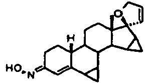 | (E/Z)-3-(Hydroxyimino)-6α,7α;15α,16α-bismethylen-17α-(1'-propenyl)-17β-3'-oxidoestra-4-en |
| | (E/Z)-3-(Hydroxyimino)-6β,7β;15α,16α-bismethylen-17α-(1'-propenyl)-17β-3'-oxidoestra-4-en |
| | (E/Z)-3-(Hydroxyimino)-6α,7α;15β,16β-bismethylen-17α-(1'-propenyl)-17β-3'-oxidoestra-4-en |
| | (E/Z)-3-(Hydroxyimino)-6β,7β;15β,16β-bismethylen-17α-(1'-propenyl)-17β-3'-oxidoestra-4-en |
| 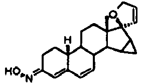 | (E/Z)-3-(Hydroxyimino)-17α-(1'-propenyl)-15α,16α-methylen-17β-3'-oxidoestra-4,6-dien |
| | (E/Z)-3-(Hydroxyimino)-17α-(1'-propenyl)-15β,16β-methylen-17β-3'-oxidoestra-4,6-dien |
| | |
| 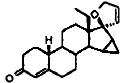 | 17α-(1'-Propenyl)-18-methyl-15α,16α-methylen-17β-3'-oxidoestra-4-en-3-on |
| | 17α-(1'-Propenyl)-18-methyl-15β,16β-methylen-17β-3'-oxidoestra-4-en-3-on |
| 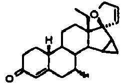 | 7α,18-Dimethyl-15α,16α-methylen-17α-(1'-propenyl)-17β-3'-oxidoestra-4-en-3-on |
| | 7α,18-Dimethyl-15β,16β-methylen-17α-(1'-propenyl)-17β-3'-oxidoestra-4-en-3-on (Beispiel 4A) |
| | 7β,18-Dimethyl-15α,16α-methyten-17α-(1'-propenyl)-17β-3'-oxidoestra-4-en-3-on |
| | 7β,18-Dimethyl-15β,16β-methylen-17a-(1'-propenyl)-17β-3'-oxidoestra-4-en-3-on |
| 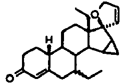 | 7a-Ethyl-18-methyl-15α,16α-methylen-17α-(1'-propenyl)-17β-3'-oxidoestra-4-en-3-on |
| | 7α-Ethyl-18-methyl-15β,16β-methylen-17α-(1'-propenyl)-17β-3'-oxidoestra-4-en-3-on (Beispiel 5A) |
| | 7β-Ethyl-18-methyl-15α,16α-methylen-17α-(1'-propenyl)-17β-3'-oxidoestra-4-en-3-on |
| | 7β-Ethyl-18-methyl-15β,16β-methylen-17a-(1'-propenyl)-17β-3'-oxidoestra-4-en-3-on (Beispiel 5B) |
| 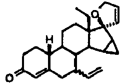 | 7α-Vinyl-18-methyl-15α,16α-methylen-17α-(1'-propenyl)-17β-3'-oxidoestra-4-en-3-on |
| | 7α-Vinyl-18-methyl-17αβ,16β-methylen-17a-(1'-propenyl)-17β-3'-oxidoestra-4-en-3-on (Beispiel 6A) |
| | 7β-Vinyl-18-methyl-15α,16α-methylen-17α-(1'-propenyl)-17β-3'-oxidoestra-4-en-3-on |
| | 7β-Vinyl-18-methyl-15β,16β-methylen-17α-(1'-propenyl)-17β-3'-oxidoestra-4-en-3-on |
| 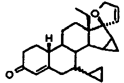 | 7α-Cycloprypyl-18-methyl-15α,16α-methylen-17α-(1'-propenyl)-17β-3'-oxidoestra-4-en-3-on |
| | 7α-Cycloprypyl-18-methyl-15β,16β-methylen-17α-(1'-propenyl)-17β-3'-oxidoestra-4-en-3-on (Beispiel 7A) |
| | 7β-Cydopropyl-18-methyl-15α,16α-methylen-17α-(1'-propenyl)-17β-3'-oxidoestra-4-en-3-on |
| | 7β-Cyclopropyl-18-methyl-15β,16β-methylen-17α-(1'-propenyl)-17β-3'-oxidoestra-4-en-3-on |
| 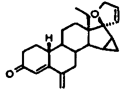 | 6-Methylen-18-methyl-15α,16α-methylen-17α-(1'-propenyl)-17β-3'-oxidoestra-4-en-3-on |
| | 6-Methylen-18-methyl-15β,16β-methylen-17α-(1'-propenyl)-17β-3'-oxidoestra-4-en-3-on |
| 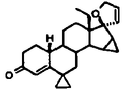 | 6,6-(1,2-Ethandiyl)-18-methyl-15α,16α-methylen-17α-(1'-propenyl)-17β-3'-oxidoestra-4-en-3-on |
| | 6,6-(1,2-Ethandiyl)-18-methyl-15β,16β-methylen-17α-(1'-propenyl)-17β-3'-oxidoestra-4-en-3-on (Beispiel 10) |
| 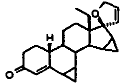 | 6α,7α;15α,16α-Bismethylen-18-methyl-17α-(1'-propenyl)-17β-3'-oxidoestra-4-en-3-on |
| | 6β,7β;15α,17α-Bismethylen-18-methyl-17βα-(1'-propenyl)-17β-3'-oxidoestra-4-en-3-on |
| | 6α,7βα;15β,16β-Bismethylen-18-methyl-17βα-(1'-propenyl)-17β-3'-oxidoestra-4-en-3-on (Beispiel 2) |
| | 6β,7β;15β,16β-Bismethylen-18-methyl-17βα-(1'-propenyl)-17β-3'-oxidoestra-4-en-3-on (Beispiel 1) |
| 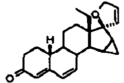 | 17α-(1'-Propenyl)-18-methyl-15α,16α-methylen-17β-3'-oxidoestra-4,6-dien-3-on |
| | 17α-(1'-Propenyl)-18-methyl-15β,16β-methylen-17β-3'-oxidoestra-4,6-dien-3-on (Beispiel 3) |
| | |
| 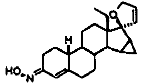 | (E/Z)-3-(Hydroxyimino)-18-methyl-17a-(1'-propenyl)-15α,16α-methylen-17β-3'-oxidoestra-4-en |
| | (E/Z)-3-(Hydroxyimino)-18-methyl-17α-(1'-propenyl)-15β,16β-methylen-17β-3'-oxidoestra-4-en |
| 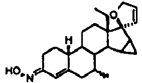 | (E/Z)-3-(Hydroxyimino)-7α,18-bismethyl-15α,16α-methylen-17α-(1'-propenyl)-17β-3'-oxidoestra-4-en |
| | (E/Z)-3-(Hydroxyimino)-7α,18-bismethyl-15β,16β-methy(en-17α-(1'-propenyl)-17β-3'-oxidoestra-4-en |
| | (E/Z)-3-(Hydroxyimino)-7β,18-bismethyl-15α, 16α-methylen-17α-(1'-propenyl)-17β-3'-oxidoestra-4-en |
| | (E/Z)-3-(Hydroxyimino)-7β,18-bismethyl-15β,16β-methylen-17α-(1'-propenyl)-17β-3'-oxidoestra-4-en |
| 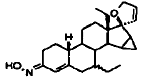 | (E/Z)-3-(Hydroxyimino)-7α-ethyl-18-methyl-15α,16α-methylen-17α-(1'-propenyl)-17β-3'-oxidoestra-4-en |
| | (E/Z)-3-(Hydroxyimino)-7α-ethyl-18-methyl-15β,16β-methylen-17α-(1'-propenyl)-17β-3'-oxidoestra-4-en |
| | (E/Z)-3-(Hydroxyimino)-7β-ethyl-18-methyl-15α,16α-methylen-17α-(1'-propenyl)-17β-3'-oxidoestra-4-en |
| | (E/Z)-3-(Hydroxyimino)-7β-ethyl-18-methyl-15β,16β-methylen-17α-(1'-propenyl)-17β-3'-oxidoestra-4-en |
| 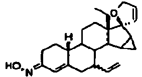 | (E/Z)-3-(Hydroxyimino)-7α-vinyl-18-methyl-15α,16α-methylen-17α-(1'-propenyl)-17β-3'-oxidoestra-4-en |
| | (E/Z)-3-(Hydroxyimino)-7α-vinyl-18-methyl-15β,16β-methylen-17α-(1'-propenyl)-17β-3'-oxidoestra-4-en |
| | (E/Z)-3-(Hydroxyimino)-7β-vinyl-18-methyl-15α,16α-methylen-17α-(1'-propenyl)-17β-3'-oxidoestra-4-en |
| | (E/Z)-3-(Hydroxyimino)-7β-vinyl-18-methyl-15β,16β-methylen-17α-(1'-propenyl)-17β-3'-oxidoestra-4-en |
| 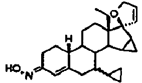 | (E/Z)-3-(Hydroxyimino)-7α-cyclopropyl-18-methyl-15α,16α-methylen-17α-(1'-propenyl)-17β-3'-oxidoestra-4-en |
| | (E/Z)-3-(Hydroxyimino)-7α-cyclopropyl-18-methyl-15β,16β-methylen-17α-(1'-propenyl)-17β-3'-oxidoestra-4-en |
| | (E/Z)-3-(Hydroxyimino)-7β-cyclopropyl-18-methyl-15α,16α-methylen-17α-(1'-propenyl)-17β-3'-oxidoestra-4-en |
| | (E/Z)-3-(Hydroxyimino)-7β-cyclopropyl-18-methyl-15β,16β-methylen-17α-(1'-propenyl)-17β-3'-oxidoestra-4-en |
| 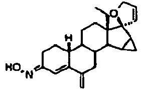 | (E/Z)-3-(Hydroxyimino)-18-methyl-6-methylen-17α-(1'-propenyl)-15α,16α-methylen-17β-3'-oxidoestra-4-en |
| | (E/Z)-3-(Hydroxyimino)-18-methyl-6-methylen-17α-(1'-propenyl)-15β,16β-methylen-17β-3'-oxidoestra-4-en |
| 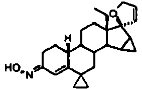 | (E/Z)-3-(Hydroxyimino)-6,6-(1,2-ethandiyl)-17α-(1'-propenyl)-18-methyl-15α,16α-methylen-17β-3'-oxidoestra-4-en |
| | (E/Z)-3-(Hydroxyimino)-6,6-(1,2-ethandiyl)-17α-(1'-propenyl)-18-methyl-15β,16β-methylen-17β-3'-oxidoestra-4-en |
| 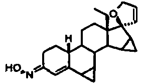 | (E/Z)-3-(Hydroxyimino)-6α,7α;15α,16α-bismethylen-18-methyl-17α-(1'-propenyl)-17β-3'-oxidoestra-4-en |
| | (E/Z)-3-(Hydroxyimino)-6β,7β;15α,16α-bismethylen-18-methyl-17α-(1'-propenyl)-17β-3'-oxidoestra-4-en |
| | (E/Z)-3-(Hydroxyimino)-6α,7α;16β,16β-bismethylen-18-methyl-17α-(1'-propenyl)-17β-3'-oxidoestra-4-en |
| | (E/Z)-3-(Hydroxyimino)-6β,7β;15β,16β-bismethylen-18-methyl-17α-(1'-propenyl)-17β-3'-oxidoestra-4-en |
| 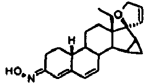 | (E/Z)-3-(Hydroxyimino)-17α-(1'-propenyl)-18-methyl-15α,16α-methylen-17β-3'-oxidoestra-4,6-dien |
| | (E/Z)-3-(Hydroxyimino)-17α-(1'-propenyl)-18-methyl-15β,16β-methylen-17β-3'-oxidoestra-4,6-dien |

Aufgrund ihrer gestagenen Wirksamkeit können die neuen Verbindungen mit der allgemeinen chemischen Formel I allein oder in Kombination mit Estrogen in Arzneimitteln zur Kontrazeption verwendet werden.

Die erfindungsgemäßen Derivate eignen sich daher insbesondere zur Herstellung eines Arzneimittels zur oralen Kontrazeption und zur Behandlung von prä-, peri- und postmenopausalen Beschwerden, einschließlich der Verwendung in Präparaten für die Hormon-Substitutionstherapie (HRT).

Wegen ihres günstigen Wirkungsprofils sind die erfindungsgemäßen Derivate außerdem besonders gut geeignet zur Behandlung prämenstrueller Beschwerden, wie Kopfschmerzen, depressiver Verstimmungen, Wasserretention und Mastodynie.

Besonders bevorzugt ist die Verwendung der erfindungsgemäßen Derivate zur Herstellung eines Arzneimittels mit gestagener, bevorzugt auch antimineralcorticoider und neutraler bis leicht androgener Wirkung.

Eine Behandlung mit den erfindungsgemäßen Derivaten findet bevorzugt am Menschen statt, kann aber auch an verwandten Säugetierspezies, wie beispielsweise an Hund und Katze, durchgeführt werden.

Zur Verwendung der erfindungsgemäßen Derivate als Arzneimittel werden diese mit mindestens einem geeigneten pharmazeutisch unbedenklichen Zusatzstoff, beispielsweise Trägerstoff, kombiniert. Der Zusatzstoff ist beispielsweise für die parenterale, vorzugsweise orale, Applikation geeignet. Es handelt sich dabei um pharmazeutisch geeignete organische oder anorganische inerte Zusatzmaterialien, wie zum Beispiel, Wasser, Gelatine, Gummi arabicum, Milchzucker, Stärke, Magnesiumstearat, Talk, pflanzliche Öle, Polyalkylenglykole usw. Die Arzneimittel können in fester Form, zum Beispiel als Tabletten, Dragees, Suppositorien, Kapseln oder in flüssiger Form, zum Beispiel als Lösungen, Suspensionen oder Emulsionen, vorliegen. Gegebenenfalls enthalten sie darüber hinaus Hilfsstoffe, wie Konservierungs-, Stabilisierungs-, Netzmittel oder Emulgatoren, Salze zur Veränderung des osmotischen Druckes oder Puffer. Für die parenterale Applikation sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden. Es ist auch möglich, die erfindungsgemäßen Derivate in ein transdermales System einzuarbeiten und sie damit transdermal zu applizieren. Für die orale Applikation kommen insbesondere Tabletten, Dragees, Kapseln, Pillen, Suspensionen oder Lösungen in Frage.

Als Applikationswege kommen weiterhin beispielsweise eine intravaginale oder intrauterine Gabe in Frage. Diese kann durch physiologisch verträgliche Lösungen, wie z.B. einer wässrigen oder öligen Lösung mit oder ohne geeigneten Löslichkeitsvermittler, Dispersionsmittel oder Emulgatoren erfolgen. Als geeignete Öle kommen beispielsweise Erdnussöl, Baumwollsamenöl, Rizinusöl oder Sesamöl in Frage. Die Auswahl ist damit keineswegs darauf beschränkt.

Für die intravaginale oder intrauterine Gabe können spezielle Systeme wie ein intravaginales System (z.B. Vaginalring, VRS) oder ein intrauterines System (IUS) verwendet werden, die eine aktive Substanz der vorliegenden Erfindung aus einem Reservoir auch über eine längere Zeit (z. B. 1, 2, 3, 4 oder 5 Jahre) freisetzen.

Als Beispiel für ein intrauterines System sei stellvertretenderweise MIRENA® angeführt. Hierbei handelt es sich um ein T-förmiges, Levonorgestrel freisetzendes intrauterines Sytem der BAYER SCHERING PHARMA AG.

Weiterhin kann eine Applikation über ein implantiertes Depotsystem aus einem inerten Trägermaterial wie z.B. einem biologisch abbaubaren Polymer oder einem synthetischen Silikon-Polymer erfolgen. Diese Depotsysteme setzten den Wirkstoff kontrolliert über einen längeren Zeitraum frei (z.B. 3 Monate bis 3 Jahre) und werden subkutan implantiert.

Die Dosierung der erfindungsgemäßen Derivate in Kontrazeptionspräparaten soll 0,01 bis 10 mg pro Tag betragen. Die Tagesdosis bei der Behandlung prämenstrueller Beschwerden liegt bei etwa 0,1 bis 20 mg. Die erfindungsgemäßen gestagenen Derivate werden in Kontrazeptionspräparaten sowie in den Arzneimitteln zur Behandlung prämenstrueller Beschwerden vorzugsweise oral appliziert. Die tägliche Dosis wird vorzugsweise einmalig verabreicht. Die vorstehend genannten Dosierungen beziehen sich auf orale Verabreichungsformen.

Bei Verwendung einer Depotformulierung wird die entsprechende, zu den vorstehend genannten oralen Dosierungen equivalente Dosierung kontinuierlich pro Tag aus den längerfristig eingesetzten oben beschriebenen Depotsystemen freigesetzt.

Aus einer Depotformulierung, zum Beispiel aus einem IUS, wird täglich eine Menge von 0,005 bis 10 mg einer Verbindung der allgemeinen Formel 1 freigesetzt.

Die gestagenen und estrogenen Wirkstoffkomponenten werden in Kontrazeptionspräparaten vorzugsweise zusammen oral appliziert. Die tägliche Dosis wird vorzugsweise einmalig verabreicht.

Als Estrogene kömmen synthetische Estrogene, vorzugsweise Ethinylestradiol, aber auch Mestranol, sowie natürliche Estrogene, einschließlich Phytoestrogene, in Betracht.

Das Estrogen wird in einer täglichen Menge verabreicht, die der pharmakologischen Wirkung von 0,01 bis 0,04 mg Ethinylestradiol entspricht. Diese Menge bezieht sich auf eine orale Verabreichungsform. Wird eine anderer Verabreichungsweg gewählt ist eine entsprechende, zur vorstehend genannten oralen Dosierung equivalente Dosierungsmenge zu verwenden.

Als Estrogene in den Arzneimitteln zur Behandlung von prä-, peri- und postmenopausalen Beschwerden sowie für die Hormon-Substitutionstherapie kommen in erster Linie natürliche Estrogene zur Anwendung, vor allem das Estradiol, aber auch die Ester von Estradiol, beispielsweise Estradiolvalerat, oder auch konjugierte Estrogene (CEEs = Conjugated Equine Estrogens).

Die gestagene, antimineralcorticoide und androgene bzw. antiandrogene Wirkung der erfindungsgemaßen Verbindungen wurde mit den folgenden Methoden untersucht:

### 1. Progesteronrezeptor-Bindungstest:

Unter Verwendung von Cytosol aus Progesteronrezeptor-exprimierenden Insektenzellen (Hi5) wurde die kompetitiive Bindefähigkeit an den Progesteronrezeptor ermittelt über die Fähigkeit, ³H-Progesteron als Bezugssubstanz vom Rezeptor zu verdrängen. Verfügt eine Verbindung über eine Progesteron entsprechende Affinität, entspricht das dem Kompetitionsfaktor (KF) von 1. KF-Werte größer als 1 zeichnen sich durch eine geringere, KF-Werte kleiner als 1 durch eine höhere Affinität zum Progesteronrezeptor aus.

### 2. Mineralocorticoidrezeptor-Bindungstest:

Der Test erfolgte analog zu 1., mit folgenden Modifikationen: Zum Einsatz kam Cytosol aus Mineralocorticoidrezeptor-exprimierenden Insektenzellen (Hi5), die Bezugssubstanz war ³H-Aldosteron.

### 3. Androgenrezeptor-Bindungstest:

Der Test erfolgte analog zu 1., mit folgenden Modifikationen: Zum Einsatz kam Cytosol aus Androgenrezeptor-exprimierenden Insektenzellen (Hi5), die Bezugssubstanz war ³H-Testosteron.

Die Ergebnisse der Bindungstests sowie das Verhältnis der Kompetitionsfaktoren KF(PR) und KR(MR) sind in Tabelle 1 wiedergegeben, wobei zum Vergleich Rezeptorbindungswerte auch von Drospirenon als Bezugssubstanz A angegeben sind.

### 4. Bestimmung der gestagenen Wirkung mithilfe von Transaktivierungstests:

Zur Kultivierung der für den Assay verwendeten Zellen wurde als Kultivierungsmedium DMEM (Dulbecco's Modified Eagle Medium: 4500 mg/ml Glukose; PAA, #E15-009) mit 10% FCS (Biochrom, S0115, Charge #615B), 4 mM L-Glutamin, 1 % Penicillin/Streptomycin, 1 mg/ml G418 und 0,5 µg/ml Puromycin verwendet.

Reporter-Zelllinien (CHO K1 Zellen stabil transfiziert mit einem Fusionsprotein aus der PR-Ligandenbindungsdomäne und einer Gal4-Transaktivierungsdomäne sowie einem Reporterkonstrukt, das die Luciferase unter der Kontrolle eines Gal4-responsiven Promotors enthielt) wurden in einer Dichte von 4 x 10⁴ Zellen pro Vertiefung in weißen, undurchsichtigen Gewebekulturplatten mit jeweils 96 Vertiefungen angezüchtet (PerkinElmer, #P12-106-017) und in Kultivierungsmedium mit 3 % DCC-FCS (Aktivkohle behandeltes Serum, zur Entfernung im Serum enthaltener störender Komponenten) gehalten. Die zu untersuchenden Verbindungen wurden acht Stunden später zugegeben, und die Zellen wurden mit den Verbindungen 16 Stunden lang inkubiert. Die Versuche wurden dreifach ausgeführt. Am Ende der Inkubation wurde das Effektor enthaltende Medium entfernt und durch Lysis-Puffer ersetzt. Nachdem Luciferase-Assay-Substrat (Promega, #E1501) zugegeben worden war, wurden die Platten mit den 96 Vertiefungen dann in ein Mikroplatten-Luminometer (Pherastar, BMG labtech) eingeführt, und die Lumineszenz wurde gemessen. Die IC₅₀-Werte wurden unter Verwendung einer Software zur Berechnung von Dosis-Wirkungsbeziehungen ausgewertet. In Tabelle 2 sind Versuchsergebnisse und zum Vergleich entsprechende Ergebnisse von Drospirenon als Bezugssubstanz A wiedergegeben.

### 5. Bestimmung der antimineralocorticoiden Wirkung mithilfe von Transaktivierungstests:

Die Bestimmung der antimineralocorticoiden Aktivität der Testsubstanzen erfolgte analog zu den oben beschriebenen Transaktivierungstests.

Folgende Modifikationen wurden vorgenommen: Hier kamen Reporterzellinien zum Einsatz (MDCK Zellen), die den humanen Mineralocorticoidrezeptor exprimieren, sowie transient ein Reporterkonstrukt enthalten, das Luciferase unter der Kontrolle eines steroidhormon-responsiven Promoters enthält.

Zur Kultivierung der für den Assay verwendeten Zellen wurde als Kultivierungsmedium DMEN EARLE'S MEM (PAA, Cat.: E 15-025) versehen mit 100U Penicillin / 0,1mg/ml Streptomycin (PAA, Cat: P11-010), 4mM L-Glutamin (PAA, Cat: M11-004) sowie foetalem Kälberserum (BIO Witthaker, Cat: DE14-801F) verwendet.

Zur Bestimmung der antimineralocorticoiden Wirksamkeit wurde den Zellen 1 nM Aldosteron (SIGMA A-6628, Lot 22H4033) zugesetzt, um eine fastmaximale Stimulation des Reportergens zu erreichen. Eine Inhibition des Effektes zeigte eine mineralcorticoidantagonistische Wirkung der Substanzen an (Tabelle 2; zum Vergleich entsprechende Werte für Drospirenon (A)).

### 6. Bestimmung der androgenen / antiandrogenen Wirkung mithilfe von Transaktivierungstests:

Die Bestimmung der androgenen / antiandrogenen Wirkung der Testsubstanzen erfolgte analog zu den oben beschriebenen Transaktivierungstests.

Folgende Modifikationen wurden vorgenommen: Hier kamen Reporterzelllinien zum Einsatz (PC3 Zellen), die den Androgenrezeptor exprimieren, sowie ein Reporterkonstrukt, das Luciferase unter der Kontrolle eines steroidhormon-responsiven Promoters enthält.

Zur Kultivierung der für den Assay verwendeten Zellen wurde als Kultivierungsmedium RPMI Medium ohne Phenolrot (PAA, #E15-49), versehen mit 100U Penicillin / 0,1 mg/ml Streptomycin (PAA, Cat: P11-010), 4 mM L-Glutamin (PAA, Cat: M11-004) sowie foetalem Kälberserum (BIO Witthaker, Cat: DE14-801F), verwendet.

Zur Bestimmung der antiandrogenen Wirksamkeit wurde den Zellen 0,05 nM R1881 zugesetzt, um eine fastmaximale Stimulation des Reportergens zu erreichen. Eine Inhibition des Effektes zeigte eine androgen-antagonistische Wirkung der Substanzen an (Tabelle 2; zum Vergleich entsprechende Werte für Drospirenon (A)).

Soweit die Herstellung der Ausgangsverbindungen hier nicht beschrieben ist, sind diese dem Fachmann bekannt oder analog zu bekannten Verbindungen oder hier beschriebenen Verfahren herstellbar. Die Isomerengemische können nach üblichen Methoden, wie beispielsweise Kristallisation, Chromatographie oder Salzbildung, in die einzelnen Verbindungen aufgetrennt werden. Die Herstellung der Salze erfolgt in üblicher Weise, indem man eine Lösung der Verbindungen mit der allgemeinen chemischen Formel I mit der äquivalenten Menge oder einem Überschuss einer Base oder Säure, die sich gegebenenfalls in Lösung befindet, versetzt, gegebenenfalls den Niederschlag abtrennt oder in üblicher Weise die Lösung aufarbeitet.

Die Darstellung der Verbindungen mit der allgemeinen chemischen Formel I, ausgehend von Verbindungen mit der allgemeinen chemischen Formel 1 (Schema 2), erfolgt nach den in Schema 1 angegebenen Verfahren, worin R⁴, R^{6a}, R^{6b}, R⁷, R¹⁸ und Z die vorgenannten Bedeutungen haben, und
- R⁶, R⁷: in 5 und 6 gemeinsam Sauerstoff oder eine Methylengruppe bilden,
- U: Sauerstoff, zwei Alkoxygruppen OR¹⁹, eine C₂-C₁₀Alkylen-α,ω-dioxygruppe, die geradkettig oder verzweigt sein kann, ist, wobei R¹⁹ für einen C₁-C₂₀-Alkylrest steht,
- R²⁰: ein C₁-C₂₀-Alkylrest ist,
- X: eine NR^{21a}R^{21b}-Gruppe oder eine Alkoxygruppe OR²² ist,
- R^{21a}, R^{21b}: jeweils unabhängig voneinander ausgewählt sind aus der Gruppe, umfassend Wasserstoff und C₁-C₁₀-Alkyl, oder gemeinsam eine C₄-C₁₀-α,ω-Alkylengruppe bilden, die geradkettig oder verzweigt sein kann und
- R²²: ein C₁-C₂₀-Alkylrest ist.

Die Verbindungen **2** und **3** in Schema 1 tragen jeweils eine Doppelbindung zwischen C⁵ und C⁶ oder zwischen C⁵ und C¹⁰ sowie eine weitere Doppelbindung zwischen C² und C³ oder zwischen C³ und C⁴.

Die Verbindungen **7** bis **9** in Schema 1 tragen jeweils eine Doppelbindung zwischen C⁴ und C⁵ oder zwischen C⁵ und C⁶ oder zwischen C⁵ und C¹⁰.

Für den Fachmann ist es selbstverständlich, dass bei den Beschreibungen der synthetischen Transformationen immer vorausgesetzt wird, dass gegebenenfalls am Steroidgerüst vorhandene sonstige funktionelle Gruppen in geeigneter Form geschützt sind.

Die Einführung einer 6,7-Doppelbindung unter Bildung von Verbindungen mit den allgemeinen chemischen Formeln **4**, **13** oder **18** erfolgt über Bromierung der jeweiligen 3,5-Dienolether **3**, **12** oder **17** sowie anschließende Bromwasserstoffabspaltung (siehe z. B. J. Fried, J.A. Edwards, Organic Reactions in Steroid Chemistry, von Nostrand Reinhold Company 1972, S. 265-374).

Die Dienoletherbromierung der Verbindungen **3**, **12** oder **17** kann beispielsweise analog der Vorschrift aus Steroids 1, 233 (1963) erfolgen. Die Bromwasserstoffabspaltung unter Bildung der Verbindungen mit den allgemeinen chemischen Formeln **4**, **13** oder **18** gelingt durch Erhitzen der 6-Bromverbindung mit basischen Reagenzien, wie beispielsweise LiBr oder Li₂CO₃ in aprotischen Lösungsmitteln, wie Dimethylformamid, bei Temperaturen von 50-120°C oder aber indem die 6-Bromverbindungen in einem Lösungsmittel, wie Collidin oder Lutidin, erhitzt werden.

Die Einführung eines Substituenten R⁴ kann zum Beispiel, ausgehend von einer Verbindung mit einer der allgemeinen chemischen Formeln **6**, **11**, **13**, **14**, **16** oder **18** durch Epoxidierung der 4,5-Doppelbindung mit Wasserstoffperoxid unter alkalischen Bedingungen und Umsetzung der entstandenen Epoxide in einem geeigneten Lösungsmittel mit Säuren mit der allgemeinen chemischen Formel H-R⁴ erfolgten, wobei R⁴ ein Halogenatom, vorzugsweise Chlor oder Brom, sein kann. Verbindungen, in denen R⁴ die Bedeutung von Brom besitzt, lassen sich beispielsweise mit 2,2-Difluor-2-(fluorsulfonyl)essigsäuremethylester in Dimethylformamid in Gegenwart von Kupfer(I)iodid zu Verbindungen umsetzen, in denen R⁴ die Bedeutung Fluor besitzt. Alternativ kann Halogen, ausgehend von einer Verbindung mit einer der allgemeinen chemischen Formeln **6**, **11**, **13**, **14**, **16** oder **18**, durch Umsetzung mit Sulfurylchlorid oder Sulfurylbromid in Gegenwart einer geeigneten Base, wie beispielsweise Pyridin, mit R⁴ in der Bedeutung Chlor oder Brom direkt eingeführt werden.

Verbindung **4** wird durch Methenylierung der 6,7-Doppelbindung nach bekannten Verfahren beispielsweise mit Dimethylsulfoxoniummethylid (siehe z. B. DE-A 11 83 500, DE-A 29 22 500, EP-A 0 019 690, US-A 4,291,029; J. Am. Chem. Soc. 84, 867 (1962)) in eine Verbindung **5** (R⁶, R⁷ gemeinsam eine Methylengruppe) umgewandelt, wobei ein Gemisch der α- und β-Isomeren erhalten wird, das beispielsweise durch Chromatographie in die einzelnen Isomeren getrennt werden kann.

Verbindungen vom Typ **5** können, wie in den Beispielen beschrieben oder analog zu diesen Vorschriften, unter Verwendung analoger zu den dort beschriebenen Reagenzien erhalten werden.

Die Synthese der spirocyclischen Verbindung **18** (R^{6a}, R^{6b} bilden gemeinsam 1,2-Ethandiyl) geht von Verbindung **11** oder **14** aus, welche zunächst in ein 3-Amino-3,5-dien-Derivat **15** (X = NR^{21a}R^{21b}) überführt wird. Durch Umsetzung mit Formalin in alkoholischer Lösung wird das 6-Hydroxymethylen-Derivat **16** (R⁶ = Hydroxymethylen) erhalten. Nach Überführung der Hydroxygruppe in eine Fluchtgruppe, wie etwa ein Mesylat, Tosylat oder auch Benzoat, lässt sich Verbindung **18** durch Umsetzung mit Trimethylsulfoxoniumiodid unter Verwendung von Basen wie etwa Alkalihydroxiden, Alkalialkoholaten, in geeigneten Lösemitteln, wie etwa Dimethylsulfoxid, darstellen.

Zur Einführung einer 6-Methylengruppe kann Verbindung **16** (R⁶ = Hydroxymethylen) mit zum Beispiel Salzsäure in DioxanlWasser dehydratisiert werden. Auch nach Überführung der Hydroxygruppe in eine Fluchtgruppe, wie etwa ein Mesylat, Tosylat oder auch Benzoat, lässt sich Verbindung **18** (R^{6a}, R^{6b} gemeinsam Methylen) erzeugen (siehe DE-A 34 02 329, EP-A 0 150 157, US-A 4,584,288; J. Med. Chem. 34, 2464 (1991)).

Eine weitere Möglichkeit zur Herstellung von 6-Methylenverbindungen **18** besteht in der direkten Umsetzung der 4(5) ungesättigten 3-Ketone, wie Verbindung **16** (R⁶ = Wasserstoff), mit Acetalen des Formaldehyds in Gegenwart von Natriumacetat mit zum Beispiel Phosphoroxychlorid oder Phosphorpentachlorid in geeigneten Lösungsmitteln, wie Chloroform (siehe z. B. K. Annen, H. Hofmeister, H. Laurent und R. Wiechert, Synthesis 34 (1982)).

Die 6-Methylenverbindungen können zur Darstellung von Verbindungen mit der allgemeinen chemischen Formel **18**, in denen R^{6a} gleich Methyl ist und R^{6b} und R⁷ unter Bildung einer Doppelbindung zwischen C⁶ und C⁷ entfallen, genutzt werden.

Hierzu kann man beispielsweise ein in Tetrahedron 21, 1619 (1965) beschriebenes Verfahren anwenden, bei dem eine Isomerisierung der Doppelbindung durch Erwärmen der 6-Methylenverbindungen in Ethanol mit 5% Palladium-Kohle-Katalysator, der entweder mit Wasserstoff oder durch Erwärmen mit einer geringen Menge Cyclohexen vorbehandelt wurde, erzielt werden. Die Isomerisierung kann auch mit einem nicht vorbehandelten Katalysator erfolgen, wenn zur Reaktionsmischung eine geringe Menge Cyclohexen zugesetzt wird. Das Auftreten geringer Anteile hydrierter Produkte kann durch Zugabe eines Überschusses an Natriumacetat verhindert werden.

Alternativ kann die Verbindung **17** (X= OR²²) als Vorstufe verwendet werden. Die direkte Darstellung von 6-Methyl-4,6-dien-3-on-Derivaten ist beschrieben (siehe K. Annen, H. Hofmeister, H. Laurent und R. Wiechert, Lieb. Ann. 712 (1983)).

Verbindungen **18**, in denen R^{6b} eine α-Methylfunktion darstellt, können unter geeigneten Bedingungen aus den 6-Methylenverbindungen (**18**: R^{6a}, R^{6b} gemeinsam Methylen) durch Hydrierung dargestellt werden. Die besten Ergebnisse (selektive Hydrierung der exo-Methylenfunktion) werden durch Transfer-Hydrierung erreicht (J. Chem. Soc. 3578 (1954)). Erhitzt man die 6-Methylenderivate **18** in einem geeigneten Lösungsmittel, wie beispielsweise Ethanol, in Gegenwart eines Hydriddonators, wie beispielsweise Cyclohexen, so werden 6α-Methylderivate in sehr guten Ausbeuten erhalten. Geringe Anteile an 6β- Methylverbindung können sauer isomerisiert werden (Tetrahedron 1619 (1965)).

Auch die gezielte Darstellung von 6β-Methylverbindungen ist möglich. Hierfür werden die 4-En-3-one, wie etwa Verbindung **16**, beispielsweise mit Ethylenglykol, Trimethylorthoformiat in Dichlormethan in Gegenwart katalytischer Mengen einer Säure, zum Beispiel p-Toluolsulfonsäure, zu den entsprechenden 3-Ketalen umgesetzt. Während dieser Ketalisierung isomerisiert die Doppelbindung in die Position C⁵. Eine selektive Epoxidierung dieser 5-Doppelbindung gelingt beispielsweise durch Verwendung organischer Persäuren, zum Beispiel von m-Chlorperbenzoesäure, in einem geeigneten Lösungsmittel, wie Dichlormethan. Alternativ hierzu kann die Epoxidierung auch mit Wasserstoffperoxid in Gegenwart von beispielsweise von Hexachloraceton oder 3- Nitrotrifluoracetophenon erfolgen. Die gebildeten 5,6α-Epoxide können dann unter Verwendung entsprechender Alkylmagnesiumhalogenide oder Alkyllithiumverbindungen axial geöffnet werden. Auf diese Weise werden 5α-Hydroxy-6β-Alkylverbindungen erhalten. Die Spaltung der 3-Ketoschutzgruppe kann unter Erhalt der 5α-Hydroxyfunktion durch Behandeln unter milden sauren Bedingungen (Essigsäure oder 4 n Salzsäure bei 0°C) erfolgen. Basische Eliminierung der 5α-Hydroxyfunktion mit zum Beispiel verdünnter wässriger Natronlauge ergibt die 3-Keto-4-en-Verbindungen mit einer β-ständigen 6-Alkylgruppe. Alternativ hierzu ergibt die Ketalspaltung unter drastischeren Bedingungen (mit wässriger Salzsäure oder einer anderen starken Säure) die entsprechenden 6α-Alkylverbindungen.

Die Einführung einer 7-Alkyl-, 7-Alkenyl- oder 7-Alkinylgruppe unter Bildung von Verbindungen mit der allgemeinen chemischen Formel **14** erfolgt durch 1,6-Addition einer entsprechenden metallorganischen Verbindung an die Vorstufe mit der allgemeinen chemischen Formel **13** unter der Einwirkung von Kupfersalzen. Bevorzugt sind zweiwertige Metalle, wie Magnesium und Zink, als Gegenion sind Chlor, Brom und lod bevorzugt. Als Kupfersalze eignen sich ein- oder zweiwertige Kupferverbindungen, wie beispielsweise Kupferchlorid, Kupferbromid oder Kupferacetat. Die Reaktion erfolgt in einem inerten Lösungsmittel, wie beispielsweise Tetrahydrofuran, Diethylether oder Dichlormethan.

Die erhaltenen Verbindungen **6**, **11**, **13**, **14**, **16**, **18** oder **20**, in denen Z für ein Sauerstoffatom steht, können durch Umsetzung mit Hydroxylaminhydrochlorid, Alkyloxyaminhydrochloriden oder Sulfonylhydrazinen in Gegenwart eines tertiären Amins bei Temperaturen zwischen -20 und +40°C in ihre entsprechenden E/Z-konfigurierten Oxime oder Sulfonylhydrazone überführt werden (allgemeine Formel I mit Z in der Bedeutung von NOR', NNHSO₂R') Geeignete tertiäre Basen sind beispielsweise Trimethylamin, Triethylamin, Pyridin, N,N-Dimethylaminopyridin, 1,5-Diazabicyclo[4.3.0]non-5-en (DBN) und 1,5-Diazabicyclo[5.4.0]undec-5-en (DBU), wobei Pyridin bevorzugt ist. Ein analoges Verfahren ist beispielsweise in WO 98/24801 A für die Herstellung entsprechender 3-Oxyimino-Derivate des Drospirenons beschrieben.

Zur Herstellung eines Endprodukts mit der allgemeinen chemischen Formel I mit Z in der Bedeutung von zwei Wasserstoffatomen kann die 3-Oxogruppe beispielsweise nach der in DE-A 28 05 490 angegebenen Vorschrift durch reduktive Spaltung eines Thioketals der 3-Ketoverbindung auf einer geeigneten Vorstufe, wie beispielsweise von Verbindungen mit einer der allgemeinen chemischen Formeln **6**, **11**, **13**, **14**, **16**, **18** oder **20** entfernt werden.

Die Bildung von Spiroethern zu Verbindungen mit einer der allgemeinen chemischen Formeln **6** oder **11** erfolgt, ausgehend von den entsprechenden 17-Hydroxypropenylverbindungen **5** oder **10**, durch Überführung der primären Hydroxygruppe in eine Abgangsgruppe und anschließende intramolekulare Substitution. Als Abgangsgruppe eignen sich Halogenatome, wie beispielsweise Chlor, Brom oder lod, sowie Alkyl-, Aryl- oder A-ralkylsulfonate, wie beispielsweise Methansulfonat, Phenylsufonat, Tolylsulfonat, Trifluormethansulfonat, Nonafluorbutansulfonat. Die intramolekulare Zyklisierung zum Spiroether kann durch Deprotonierung der tertiären Hydroxygruppe mit geeigneten Basen, wie beispielsweise Triethylamin, Diethylamin, Diisopropylethylamin, Pyridin, Dimethylaminopyridin, Natriumhydrid, Natriumhexamethyldisilazan, Kaliumhexamethyldisilazan, Kalium-tert.-butanolat oder n-Butyllithium, erfolgen. Bevorzugt sind solche Methoden und Bedingungen, welche die Einführung der Abgangsgruppe mit unmittelbarer intramolekularer Zyklisierung in einem Reaktionsgefäß ermöglichen.

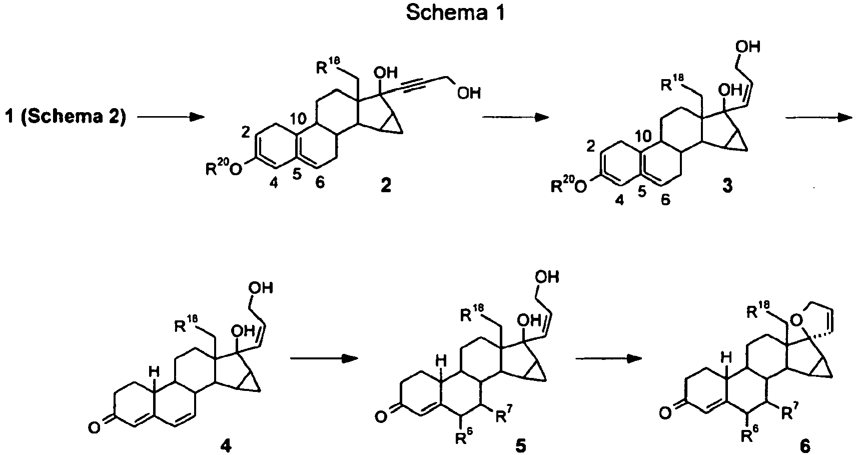

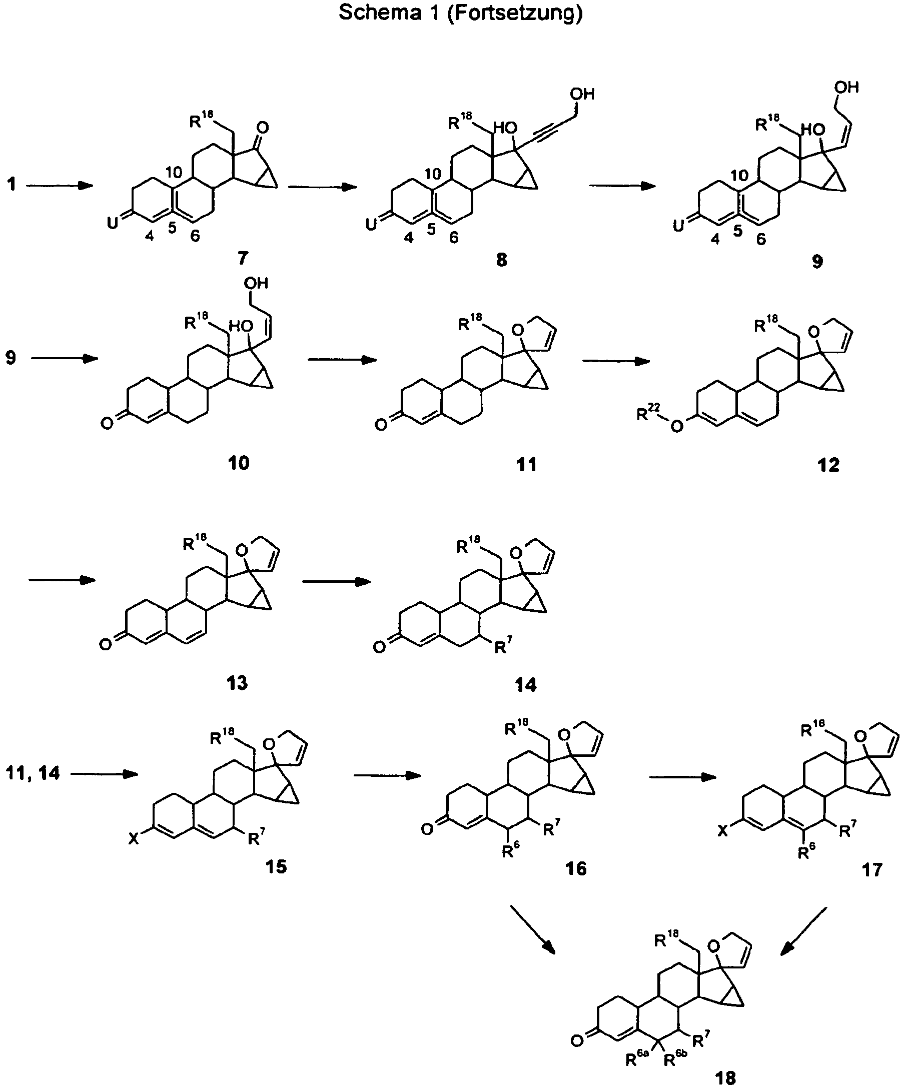

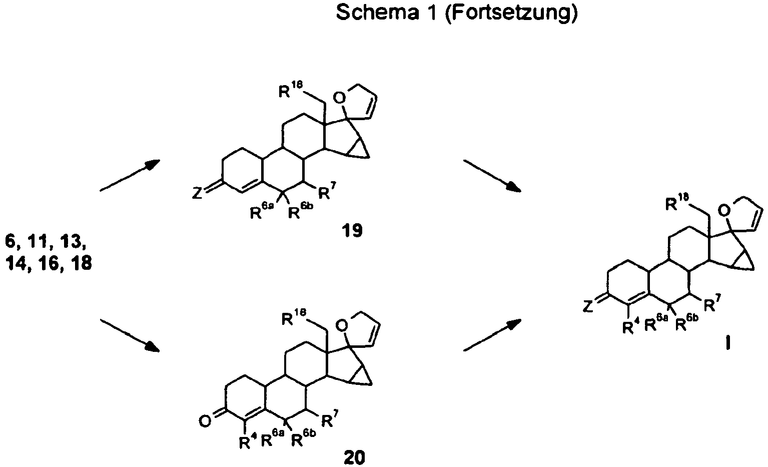

Die Verbindung **1** in Schema 2 trägt jeweils eine Doppelbindung zwischen C⁵ und C⁶ oder zwischen C⁵ und C¹⁰ sowie eine weitere Doppelbindung zwischen C² und C³ oder zwischen C³ und C4.

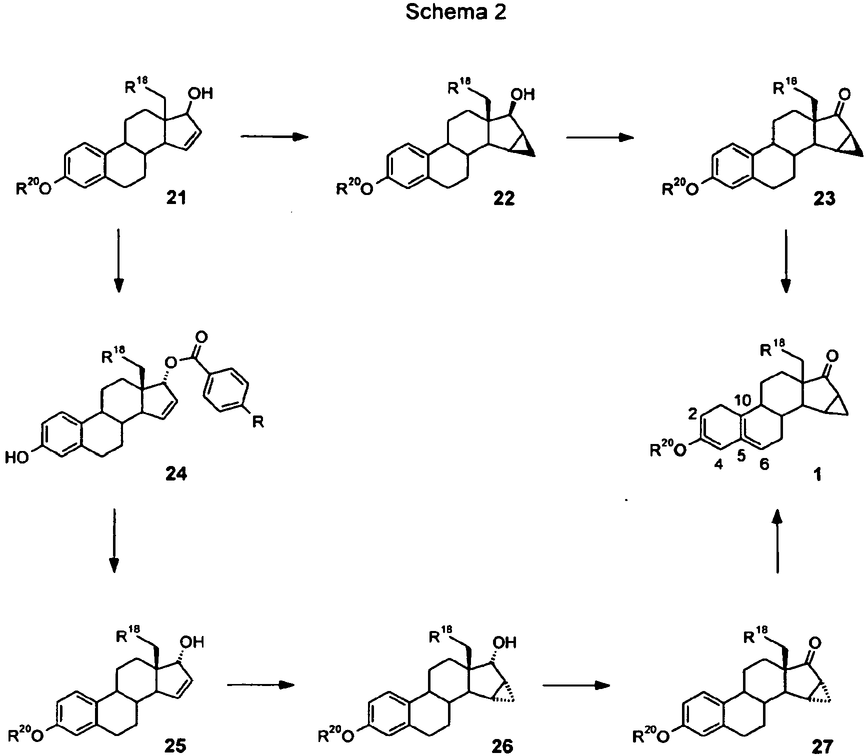

Die nachfolgenden Beispiele dienen der näheren Erläuterung der Erfindung, ohne diese auf die angeführten Beispiele einzugrenzen:

### Beispiel 1: (Spiroetherbildung)

### 6β,7β;15β,16β-Bismethylen-18-methyl-17α-(1'-propenyl)-17β-3'-oxidoestra-4-en-3-on

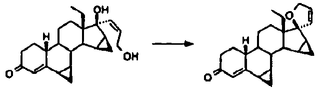

Die Lösung von 88,5 mg der nach Beispiel 1a dargestellten Verbindung A in 4,76 ml Dichlormethan versetzt man bei 3°C mit 346 µl Triethylamin, 120 mg p-Toluolsulfonsäurechlorid, lässt auf 23°C erwärmen und 15 Stunden rühren. Man gießt in gesättigte Natriumhydrogencarbonatlösung, extrahiert mehrfach mit Ethylacetat, wäscht die vereinigten organischen Extrakte mit Wasser und gesättigter Natriumchloridlösung und trocknet über Natriumsulfat. Den nach Filtration und Lösungsmittelabzug erhaltenen Rückstand reinigt man durch Chromatographie. Isoliert werden 41,1 g der Titelverbindung.
¹H-NMR (CDCl₃): δ= 0,44 (1H), 0,57 (1H), 0,84 (3H), 0,89-1,02 (3H), 1,17-1,79 (12H), 1,87 (1H), 2,02-2,19 (2H), 2,29 (1H), 2,42 (1H), 4,55 (1H), 4,71 (1H), 5,81 (1H), 5,90 (1H), 6,11 (1H) ppm.

### Beispiel 1a: (Corey Cyclopropanierung)

### 6β,7β;15β,16β-Bismethylen-17α(Z)-(3'-hydroxypropen-1'-yl)-18-methyl-17β-hydroxyestra-4-en-3-on (A) und 6α,7α;15β,16β-Bismethylen-17α(Z)-(3'-hydroxypropen-1'-yl)-18-methyl-17β-hydroxyestra-4-en-3-on (B)

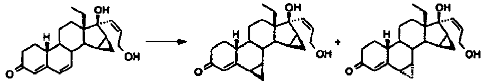

Eine Lösung von 16,8 g Sulfoxoniumiodid in 373 ml Dimethylsulfoxid versetzt man portionsweise bei 23°C mit 3,33 g einer 55%igen Suspension von Natriumhydrid in Weissöl. Man rührt noch 2 Stunden nach, versetzt mit 10,6 g der nach Beispiel 1 b dargestellten Verbindung und lässt weitere 2,5 Stunden reagieren. Man gießt in Wasser, extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt und trennt man durch Chromatographie. Isoliert werden 1,51 g der Titelverbindung A sowie 1,01 g der Titelverbindung B.

### Beispiel 1b: (Dienonbildung)

### 17α(Z)-(3'-hydroxypropen-1'-yl)-18-methyl-15β,17β-methylen-17β-hydroxyestra-4,6-dien-3-on

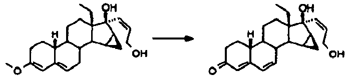

Die Lösung von 10,4 g der nach Beispiel 1c dargestellten Verbindung in 125 ml Dimethylformamid versetzt man bei 3°C mit 1,03 g Natriumacetat, 10 ml Wasser und portionsweise mit insgesamt 4,23 g Dibromhydantoin. Nach 30 Minuten versetzt man mit 3,91 g Lithiumbromid, 3,42 g Lithiumcarbonat und erhitzt 3 Stunden bei einer Badtemperatur von 100°C. Man gießt in Wasser und extrahiert mehrfach mit Ethylacetat. Die vereinigten organischen Extrakte wäscht man mit gesättigter Natriumchloridlösung und trocknet über Natriumsulfat. Den nach Filtration und Lösungsmittelabzug erhaltenen Rückstand reinigt man durch Chromatographie. Isoliert werden 6,35 g der Titelverbindung.

### Beispiel 1c: (Lindlarhydrierung)

### 17α(Z)-(3'-hydroxypropen-1'-yl)-3-methoxy-18-methyl-15β,16β-methylen-17β-hydroxy-estra-3,5-dien

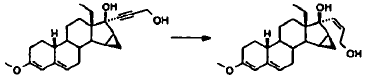

Die Lösung von 75 g der nach Beispiel 1d dargestellten Verbindung in 1,5 l Tetrahydrofuran versetzt man mit 100 ml Pyridin, 5 g Palladium auf Bariumsulfat und hydriert bei einer Atmosphäre Wasserstoff. Man filtriert über Celite und isoliert nach Einengen 75,7 g der Titelverbindung, die man ohne Reinigung weiter umsetzt.

### Beispiel 1d: (Hydroxypropinaddition)

### 17α(2)-(3'-hydroxypropin-1'-yl)-3-methoxy-18-methyl-15β,16β-methylen-17β-hydroxy-estra-3,5-dien

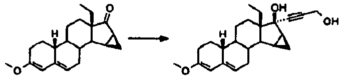

Die Lösung von 83 ml 2-Propin-1-ol in 1 I Tetrahydrofuran versetzt man bei -78°C mit 1 I einer 2,5 molaren Lösung von Buthyllithium in Hexan. Nach 30 Minuten tropft man die Lösung von 90 g 3-Methoxy-18-methyl-15β,16β-methylen-estra-3,5-dien-17-on in 0,51 Tetrahydrofuran zu, lässt auf 23°C erwärmen und rührt noch 3 Stunden. Man gießt in gesättigte, eiskalte Ammoniumchloridlösung, extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Kristallisation. Isoliert werden 90,3 g der Titelverbindung.

### Beispiel 2:

### 6α,7α;15β,16β-Bismethylen-18-methyl-17α-(1'-propenyl)-17β-3'-oxidoestra-4-en-3-on

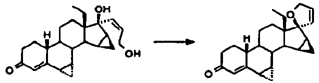

In Analogie zu Beispiel 1 setzt man 114 mg der nach Beispiel 1a dargestellten Verbindung B um und isoliert nach Aufarbeitung und Reinigung 43 mg der Titelverbindung.
¹H-NMR (CDCl₃): δ= 0,42 (1H), 0,74-1,01 (5H), 0,86 (3H), 1,19 (1H), 1,26 (1H), 1,35-1,51 (3H), 1,60-1,92 (6H), 1,98-2,15 (3H), 2,26 (1H), 2,49 (1H), 4,56 (1H), 4,71 (1H), 5,82 (1H), 5,89 (1H), 6,04 (1H) ppm.

### Beispiel 3:

### 18-Methyl-15β,16β-methylen-17α-(1'-propenyl)-17β-3'-oxidoestra-4,6-dien-3-on

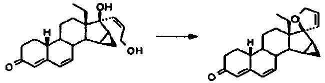

In Analogie zu Beispiel 1 setzt man 4,67 g der nach Beispiel 1a dargestellten Verbindung um und isoliert nach Aufarbeitung und Reinigung 3,66 g der Titelverbindung.
¹H-NMR (CDCl₃): δ= 0,51 (1H), 0,93 (3H), 1,01 (1H), 1,09-1,35 (4H), 1,42-1,64 (3H), 1,72-1,86 (3H), 2,00 (1H), 2,23-2,63 (5H), 4,21 (1H), 4,75 (1H), 5,84 (2H), 5,95 (1H), 6,30 (1H), 6,53 (1H) ppm.

### Beispiel 4: (1,6-Addition)

### 7α,18-Bismethyl-15β,16β-methylen-17α-(1'-propenyl)-17β-3'-oxidoestra-4-en-3-on (A) und 7β,18-Bismethyl-15β,16β-methylen-17α-(1'-propenyl)-17β-3'-oxidoestra-4-en-3-on (B)

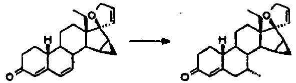

Zu der auf -30°C gekühlten Suspension von 30 mg Kupfer-(I)-chlorid in 5 ml Tetrahydrofuran tropft man 1,24 ml einer 3 molaren Lösung von Methylmagnesiumchlorid in Tetrahydrofuran und rührt noch 10 Minuten. Man kühlt auf -25°C und tropft die Lösung zu 500 mg der nach Beispiel 3 dargestellten Verbindung in 5 ml Tetrahydrofuran zu. Nach 1 Minute gießt man auf 1 N Salzsäure, extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie. Isoliert werden 267 mg der Titelverbindung A neben einem noch verunreinigten Gemisch, das Anteile der Titelverbindung B enthält.
¹H-NMR (CDCl₃) von A: δ= 0,87 (3H), 0,88 (3H), 0,90 (1H), 1,03 (1H), 1,11-1,30 (4H), 1,43-1,89 (8H), 2,05 (1H), 2,20-2,44 (5H), 2,55 (1H), 4,56 (1H), 4,71 (1H), 5,81 (1H), 5,85 (1H), 5,89 (1H) ppm.

### Beispiel 5:

### 7α-Ethyl-18-methyl-15β,16β-methylen-17α-(1'-propenyl)-17β-3'-oxidoestra-4-en-3-on (A) und 7β-Ethyl-18-methyl-15β,16β-methylen-17α-(1'-propenyl)-17β-3'-oxidoestra-4-en-3-on (B)

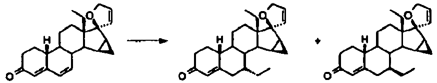

In Analogie zu Beispiel 4 setzt man 300 mg der nach Beispiel 3 dargestellten Verbindung unter Verwendung von Ethylmagnesiumchlorid um und isoliert nach Aufarbeitung und Reinigung 26 mg der Titelverbindung A sowie 26 mg der Titelverbindung B.
¹H-NMR (CDCl₃) von A: δ= 0,40 (1H), 0,87 (3H), 0,90 (1H), 0,93 (3H), 1,01 (1H), 1,06-1,17 (3H), 1,20-1,26 (2H), 1,42-1,54 (3H), 1,66 (1H), 1,71 (1H), 1,79 (1H), 1,86 (1H), 1,90 (1H), 1,95 (1H), 2,06 (1H), 2,22-2,29 (2H), 2,35-2,43 (2H), 2,60 (1H), 4,56 (1H), 4,70 (1H), 5,81 (1H), 5,86 (1H), 5,89 (1H) ppm.
¹H-NMR (CDCl₃) von B: δ= 0,45 (1H), 0,89 (3H), 0,93-1,24 (5H), 0,98 (3H), 1,33-1,89 (9H), 1,96 (1H), 2,02-2,38 (6H), 2,64 (1H), 4,56 (1H), 4,67 (1H), 5,81 (1H), 5,84 (1H), 5,91 (1H) ppm.

### Beispiel 6:

### 7α-Vinyl-18-methyl-15β,16β-methylen-17α-(1'-propenyl)-17β-3'-oxidoestra-4-en-3-on (A) und 7β-Vinyl-18-methyl-15β,16β-methylen-17α-(1'-propenyl)-17β-3'-oxidoestra-4-en-3-on (B)

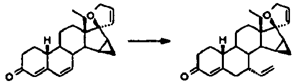

In Analogie zu Beispiel 4 setzt man 200 mg der nach Beispiel 3 dargestellten Verbindung unter Verwendung von Vinylmagnesiumchlorid um und isoliert nach Aufarbeitung und Reinigung 32 mg der Titelverbindung A neben einem noch verunreinigten Gemisch, das Anteile der Titelverbindung B enthält.
¹H-NMR (CDCl₃) von A: δ= 0,39 (1H), 0,89 (3H), 0,98 (1H), 1,04-1,33 (4H), 1,37-1,61 (3H), 1,65-1,99 (5H), 2,10-2,43 (4H), 2,54 (1H), 2,69 (1H), 2,85 (1H), 4,55 (1H), 4,67 (1H), 5,17 (1H), 5,22 (1H), 5,81-6,01 (4H) ppm.

### Beispiel 7:

### 7α-Cyclopropyl-18-methyl-15β,16β-methylen-17α-(1'-propenyl)-17β-3'-oxidoestra-4-en-3-on (A) und 7β-Cyclopropyl-18-methyl-15β,16β-methylen-17α-(1'-propenyl)-17β-3'-oxidoestra-4-en-3-on (B)

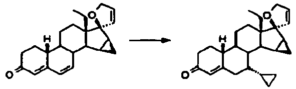

In Analogie zu Beispiel 4 setzt man 200 mg der nach Beispiel 3 dargestellten Verbindung unter Verwendung von Cyclopropylmagnesiumbromid um und isoliert nach Aufarbeitung und Reinigung 17 mg der Titelverbindung A neben einem noch verunreinigten Gemisch, das Anteile der Titelverbindung B enthält.
¹H-NMR (CDCl₃) von A: δ= 0,11 (1H), 0,31-0,75 (5H), 0,90 (3H), 0,96-1,25 (4H), 1,38-1,77 (8H), 1,84-2,03 (2H), 2,11-2,57 (6H), 4,57 (1H), 4,69 (1H), 5,84-5,97 (3H) ppm.

### Beispiel 8:

### 15β,16β-methylen-17α-(1'-propenyl)-17β-3'-oxidoestra-4-en-3-on

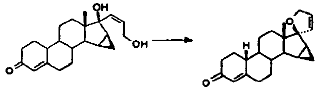

In Analogie zu Beispiel 1 setzt man 5 g der nach Beispiel 8a dargestellten Verbindung um und isoliert nach Aufarbeitung und Reinigung 3,37 g der Titelverbindung.
¹H-NMR (CD2Cl2): δ= 0,31 (1H), 0,84 (1H), 0,97 (3H), 1,05-1,32 (7H), 1,58 (2H), 1,65 (1H), 1,76 (1H), 2,03-2,41 (6H), 2,52 (1H), 4,52 (1H), 4,61 (1H), 5,77 (1H), 5,81 (1H), 5,89 (1H) ppm.

### Beispiel 8a: (Enonbildung aus Dienolether mit Oxalsäure)

### 17α(Z)-(3'-hydroxypropen-1'-yl)-15β,16β-methylen-17β-hydroxyestra-4-en-3-on

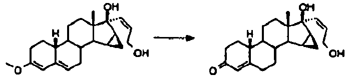

Die Suspension von 5,0 g der nach Beispiel 8b dargestellten Verbindung in 30 ml Aceton und 30 ml Wasser versetzt man mit 50 ml einer gesättigten wässrigen Lösung von Oxalsäure, gibt 30 ml Methanol und 50 ml Tetrahydrofuran zu und-rührt 5 Stunden bei 23°C. Man gießt in gesättigte Natriumhydrogencarbonatlösung, extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie. Isoliert werden 4,26 g der Titelverbindung.

### Beispiel 8b: 17α(Z)-(3'-hydroxypropen-1'-yl)-3-methoxy-15β,16β-methylen-17β-hydroxyestra-3,5-dien

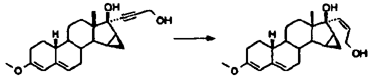

In Analogie zu Beispiel 1 c setzt man 23,8 g der nach Beispiel 8c dargestellten Verbindung um und isoliert nach Aufarbeitung und Reinigung 23,7 g der Titelverbindung.

### Beispiel 8c: 17α(Z)-(3'-hydroxypropin-1'-yl)-3-methoxy-15β,16β-methylen-17β-hydroxyestra-3,5-dien

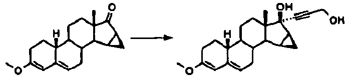

In Analogie zu Beispiel 1d setzt man 38 g 3-Methoxy-15β,16β-methylen-estra-3,5-dien-17-on um und isoliert nach Aufarbeitung und Reinigung 39,2 g der Titelverbindung.

### Beispiel 9: (6-Hydroxymethyl-Addition)

### 6β-Hydroxymethylen-18-methyl-15β,16β-methylen-17α-(1'-propenyl)-17β-3'-oxidoestra-4-en-3-on

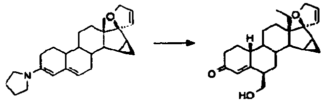

Die Lösung von 322 mg der nach Beispiel 9a dargestellten Verbindung in einem Gemisch aus 3 ml Toluol und 7 ml Ethanol versetzt man mit 323 µl einer 37%igen wässrigen Formaldehydlösung und rührt 15 Stunden bei 23°C. Man engt ein und reinigt den Rückstand durch Chromatographie. Isoliert werden 80 mg der Titelverbindung.
¹H-NMR (CDCl₃): δ= 0,38 (1H), 0,89 (3H), 0,92-1,23 (5H), 1,33 (1H), 1,40-1,85 (9H), 2,13 (1H), 2,18-2,33 (3H), 2,40 (1H), 2,71 (1H), 3,75 (2H), 4,56 (1H), 4,68 (1H), 5,85 (1H), 5,91 (1H), 5,92 (1H) ppm.

### Beispiel 9a: (Dienamin für 6-Alkylierung)

### 18-Methyl-15β,16β-methylen-3-pyrrolidinyl-17α-(1'-propenyl)-17β-3'-oxidoestra-3,5-dien

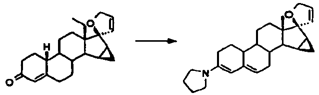

Die Lösung von 500 mg der nach Beispiel 8 dargestellten Verbindung in 5 ml Methanol versetzt man mit 282 µl Pyrrolidin und erhitzt 2 Stunden unter Rückfluss. Man kühlt ab, saugt den Niederschlag ab, wäscht mit wenig kaltem Methanol nach und erhält 329 mg der Titelverbindung, die man ohne zusätzliche Reinigung weiter umsetzt.

### Beispiel 10: (6-Spirocyclopropanierung)

### 6,6-(1,2-Ethandiyl)-18-methyl-15β,16β-methylen-17α-(1'-propenyl)-17β-3'-oxidoestra-4-en-3-on

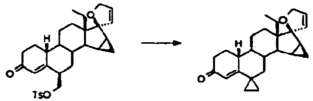

Man löst 98 mg Trimethylsulfoxoniumiodid in 1,9 ml Dimethylsulfoxid, versetzt mit 17,8 mg einer 60%igen Natriumhydrid-Dispersion und rührt 2 Stunden bei 23°C. Anschließend tropft man die Lösung von 58 mg der nach Beispiel 10a dargestellten Verbindung in 0,76 ml Dimethylsulfoxid zu und rührt weitere 2 Stunden bei 23°C. Man gießt in Wasser, extrahiert mehrfach mit Ethylacetat, wäscht die vereinigten organischen Extrakte mit Wasser und gesättigter Natriumchloridlösung und trocknet über Natriumsulfat. Den nach Filtration und Lösungsmittelabzug erhaltenen Rückstand reinigt man durch Chromatographie. Isoliert werden 15 mg der Titelverbindung.
¹H-NMR (CDCl₃): δ= 0,35 (1H), 0,45 (1H), 0,62 (1H), 0,83 (1H), 0,91 (3H), 0,95-1,35 (8H), 1,43-1,88 (7H), 1,95 (1H), 2,15-2,42 (4H), 4,56 (1H), 4,67 (1H), 5,68 (1H), 5,86 (1H), 5,92 (1H) ppm.

### Beispiel 10a: (6-Tosyloxymethyl)

### 18-Methyl-15β,16β-methylen-6β-(p-tolylsulfonyloxymethyl)-17α-(1'-propenyl)-17β-3'-oxidoestra-4-en-3-on

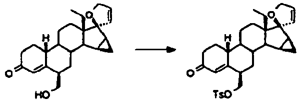

Die Lösung von 245 mg der nach Beispiel 9 dargestellten Verbindung in 11 ml Dichlormethan versetzt man mit 1,1 ml Triethylamin, 395 mg p-Toluolsulfonsäurechlorid und rührt 15 Stunden bei 23°C. Man gießt in gesättigte Natriumcarbonatlösung, extrahiert mehrfach mit Ethylacetat, wäscht die vereinigten organischen Extrakte mit Wasser und gesättigter Natriumchloridlösung und trocknet über Natriumsulfat. Den nach Filtration und Lösungsmittelabzug erhaltenen Rückstand reinigt man durch Chromatographie. Isoliert werden 58 mg der Titelverbindung.

### Beispiel 11:

### 15β,16β-methylen-17α-(1'-propenyl)-17β-3'-oxidoestra-4,6-dien-3-on

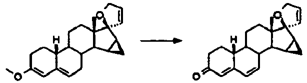

In Analogie zu Beispiel 1 b setzt man 2,5 g der nach Beispiel 11a dargestellten Verbindung um und isoliert nach Aufarbeitung und Reinigung 884 mg der Titelverbindung.
¹H-NMR (CDCl₃): δ= 0,44 (1H), 1,04 (3H), 1,11-1,61 (8H), 1,74-1,88 (2H), 2,22-2,55 (5H), 4,57 (1H), 4,67 (1H), 5,78 (1H), 5,86 (1H), 5,95 (1H), 6,30 (1H), 6,53 (1H) ppm.

### Beispiel 11a: (Dienoletherbildung)

### 15β,16β-methylen-17α-(1'-propenyl)-17β-3'-oxidoestra-4,6-dien-3-on

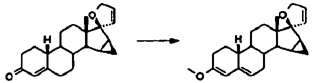

Die Lösung von 2,5 g der nach Beispiel 8 dargestellten Verbindung in 35 ml 2,2-Dimethoxypropan versetzt man mit 290 mg Pyridinium-p-toluolsulfonat und erhitzt 3 Stunden unter Rückfluss. Man gießt in gesättigte Natriumhydrogencarbonatlösung, extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand setzt man ohne Reinigung weiter um. Isoliert werden 2,6 g der Titelverbindung.

### Beispiel 12:

### 17α-(1'-Propenyl)-15α,16α-methylen-17β-3'-oxidoestra-4-en-3-on

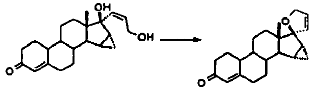

In Analogie zu Beispiel 1 setzt man 2,5 g der nach Beispiel 12a dargestellten Verbindung um und isoliert nach Aufarbeitung und Reinigung 1,66 g der Titelverbindung.
¹H-NMR (CDCl₃): δ= 0,56-0,68 (2H), 0,77 (1H), 0,97 (1H), 1,06-1,39 (5H), 1,24 (3H), 1,49-1,68 (3H), 1,77 (1H), 2,09-2,48 (6H), 2,56 (1H), 4,61 (1H), 4,70 (1H), 5,75 (1H), 5,88 (1H), 5,95 (1H) ppm.

### Beispiel 12a:

### 17α(Z)-(3'-Hydroxypropen-1'-yl)-15α,16α-methylen-17β-hydroxyestra-4-en-3-on

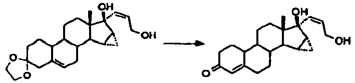

Die Lösung von 300 mg der nach Beispiel 12b dargestellten Verbindungen in 15 ml Aceton versetzt man mit 0,83 ml einer 4N Salzsäure und rührt 1 Stunde bei 23°C. Man gießt in gesättigte Natriumhydrogencarbonatlösung, extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie. Isoliert werden 135 mg der Titelverbindung.

### Beispiel 12b:

### 17α(Z)-(3'-Hydroxypropen-1'-yl)-15α,16α-methylen-17β-hydroxyestra-5-en-3-on-3-ethylenketal und 17α(Z)-(3'-Hydroxypropen-1'-yl)-15α,16α-methylen-17β-hydroxyestra-5(10)-en-3-on-3-ethylenketal

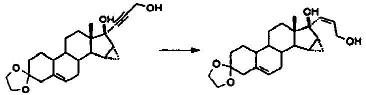

In Analogie zu Beispiel 1 c setzt man 300 mg der nach Beispiel 12c dargestellten Verbindungen um und isoliert nach Aufarbeitung 300 mg der Titelverbindungen, die man ohne Reinigung weiter umsetzt.

### Beispiel 12c:

### 17α(Z)-(3'-Hydroxypropin-1'-yl)-15α,16α-methylen-17β-hydroxyestra-5-en-3-on-3-ethylenketal und 17α(Z)-(3'-Hydroxypropin-1'-yl)-15α,16α-methylen-17β-hydroxyestra-5(1 0)-en-3-on-3-ethylenketal

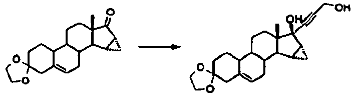

In Analogie zu Beispiel 1d setzt man 278 mg der nach Beispiel 12d dargestellten Verbindungen um und isoliert nach Aufarbeitung 347 mg der Titelverbindungen, die man ohne Reinigung weiter umsetzt.

### Beispiel 12d:

### 15α,16α-Methylen-estra-5-en-3,17-dion-3-ethylenketal und 15α,16α-methylen-estra-5(10)-en-3,17-dion-3-ethylenketal

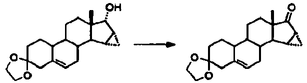

Die Lösung von 1,06 g der nach Beispiel 12e dargestellten Verbindungen in 32 ml Dichlormethan versetzt man mit einer Spatelspitze Molekularsieb 4Å, 700 mg N-Methylmorpholino-N-oxid, 90 mg Tetrabutylammoniumperruthenat und rührt bei 23°C ca. 16 Stunden. Man engt ein und reinigt den Rückstand durch Chromatographie. Isoliert werden 878 mg der Titelverbindungen.

### Beispiel 12e:

### 15α,16α-Methylen-17α-hydroxyestra-5-en-3-on-3-ethylenketal und 15α,16α(-Methylen-17α-hydroxyestra-5(110)-en-3-on-3-ethylenketal

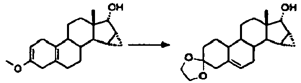

Die Lösung von 500 mg der nach Beispiel 12f dargestellten Verbindung in 10 ml Tetrahydrofuran versetzt man mit 10 ml Ethylenglykol, 4,4 mg p-Toluolsulfonsäure Hydrat und rührt bei 23°C 2 Stunden. Man gießt in gesättigte Natriumhydrogencarbonatlösung, extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie. Isoliert werden 359 mg der Titelverbindung.

### Beispiel 12f:3-Methoxy-15α,16α-methylen-17α-hydroxyestra-2,5(10)-dien

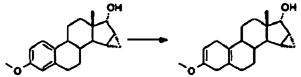

597 ml Ammoniak werden bei -75°C mit 9,91 g Lithium versetzt und innerhalb von 1 Stunde die Lösung von 24,6 g der nach Beispiel 12g dargestellten Verbindung in 1,2 l Tetrahydrofuran zugetropft. Man versetzt mit 720 ml Ethanol lässt nach 1 Stunde auf -50°C erwärmen und rührt weitere 2 Stunden. Anschließend versetzt man mit 600 ml Wasser, lässt auf 23°C erwärmen, extrahiert mehrfach mit Ethylacetat, wäscht die vereinigten organischen Extrakte mit gesättigter Natriumchloridlösung und trocknet über Natriumsulfat. Nach Filtration und Lösungsmittelabzug isoliert man 27,1 g der Titelverbindung, die ohne Reinigung weiter umgesetzt wird.

### Beispiel 12g:

### 3-Methoxy-15α,16α-methylen-17α-hydroxyestra-1,3,5(10)-trien

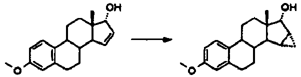

Eine Suspension aus 1,5 g Kupfer(II)acetat in 900 ml Diethylether versetzt man mit 86,6 g Zinkstaub und erhitzt 10 Minuten unter Rückfluss. Anschließend versetzt man mit 11,7 ml Diiodmethan und erhitzt weitere 30 Minuten unter Rückfluss. Man gibt die Lösung von 37,6 g der nach Beispiel 12h dargestellten Verbindung in 100 ml Tetrahydrofuran sowie über insgesamt 40 Stunden verteilt insgesamt weitere 35 ml Diiodmethan zu. Das erkaltete Gemisch filtriert man über Celite, wäscht das Filtrat mit gesättigter Natriumchloridlösung und trocknet über Natriumsulfat. Den nach Filtration und Lösungsmittelabzug erhaltenen Rückstand reinigt man durch Umkristallisation. Isoliert werden 24,6 g der Titelverbindung.

### Beispiel 12h:

### 3-Methoxy-17α-hydroxyestra-1,3,5(10),15-tetraen

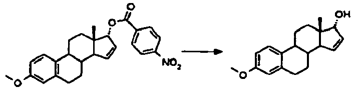

Die Lösung von 96,3 g der nach Beispiel 12i dargestellten Verbindung in 1,1 l Methanol versetzt man mit 75,5 g Kaliumcarbonat und rührt bei 50°C 2 Stunden. Man engt ein, versetzt mit Wasser, extrahiert mehrfach mit Ethylacetat, wäscht die vereinigten organischen Extrakte mit Wasser und trocknet über Natriumsulfat. Den nach Filtration und Lösungsmittelabzug erhaltenen Rückstand reinigt man durch Umkristallisation. Isoliert werden 46 g der Titelverbindung.

### Beispiel 12i:

### 4-Nitro-benzoesäure 3-Methoxy-estra-1,3,5(10),15-tetraen-17-yl ester

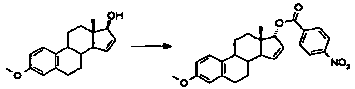

Die Lösung von 43,9 g 3-Methoxy-17β-hydroxyestra-1,3,5(10),15-tetraen in 1,6 l Tetrahydrofuran versetzt man mit 121 g Triphenylphosphin, 27,1 g 4-Nitrobenzoesäure, 30,9 ml Azodicarbonsäuredüsopropylester und rührt bei 23°C 2 Stunden. Man versetzt mit gesättigter Natriumchloridlösung, extrahiert mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand nimmt man in 1,2 l Aceton auf, versetzt unter Kühlung mit 80 ml einer 30%igen Wasserstoffperoxidlösung und gießt nach 20 Minuten unter Kühlung in 600 ml einer halbkonzentrierten Natriumthiosulfatlösung ein. Man extrahiert mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Umkristallisation. Isoliert werden 52,5 g der Titelverbindung.

### Beispiel 13:

### 17α-(1'-Propenyl)-15α,16α-methylen-17β-3'-oxidoestra-4,6-dien-3-on

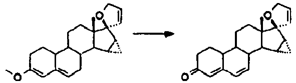

In Analogie zu Beispiel 1 b setzt man 1,13 g der nach Beispiel 13a dargestellten Verbindung um und isoliert nach Aufarbeitung und Reinigung 1,03 g der Titelverbindung.
¹H-NMR (CD₂Cl₂): δ= 0,60 (1H), 0,75 (1H), 0,90-1,74 (9H), 1,19 (3H), 2,19-2,50 (5H), 4,52 (1H), 4,62 (1H), 5,70 (1H), 5,72 (1H), 5,92 (1H), 6,23 (1H), 6,45 (1H) ppm.

### Beispiel 13a:

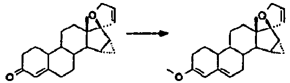

In Analogie zu Beispiel 11a setzt man 1,66 g der nach Beispiel 12 dargestellten Verbindung um und isoliert nach Aufarbeitung und Reinigung 1,13 g der Titelverbindung.

### Beispiel 14:

### 7α-Methyl-15α,16α-methylen-17α-(1'-propenyl)-17β-3'-oxidoestra-4-en-3-on (A) und 7β-Methyl-15α,16α-methylen-17α-(1'-propenyl)-17β-3'-oxidoestra-4-en-3-on (B)

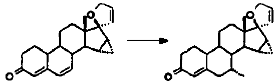

In Analogie zu Beispiel 4 setzt man 250 mg der nach Beispiel 13 dargestellten Verbindung um und isoliert nach Aufarbeitung und Reinigung 130 mg der Titelverbindung A neben einem noch verunreinigten Gemisch, das Anteile der Titelverbindung B enthält.
¹H-NMR (CD₂Cl₂) von A: δ= 0,59 (1H), 0,74 (1H), 0,87 (3H), 0,92-1,33 (6H), 1,22 (3H), 0,48-1,62 (2H), 1,76-1,88 (2H), 2,08 (1H), 2,23-2,41 (5H), 2,58 (1H), 4,56 (1H), 4,64 (1H), 5,76-5,83 (2H), 5,95 (1H) ppm.

### Beispiel 15:

### 7α-Ethyl-15α,16α-methylen-17α-(1'-propenyl)-17β-3'-oxidoestra-4-en-3-on (A) und 7β-Ethyl-15α,16α-methylen-17α-(1'-propenyl)-17β-3'-oxidoestra-4-en-3-on (B)

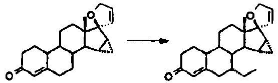

In Analogie zu Beispiel 4 setzt man 250 mg der nach Beispiel 13 dargestellten Verbindung unter Verwendung von Ethylmagnesiumchlorid um und isoliert nach Aufarbeitung und Reinigung 58 mg der Titelverbindung A sowie 5,7 mg der Titelverbindung B.
¹H-NMR (CDCl₃) von A: δ= 0,53 (1H), 0,78 (1H), 0,86-1,27 (7H), 0,90 (3H), 1,17 (3H), 1,34-1,56 (3H), 1,72-1,86 (2H), 1,93 (1H), 2,05 (1H), 2,17-2,37 (4H), 2,58 (1H), 4,52 (1H), 4,59 (1H), 5,74 (1H), 5,78 (1H), 5,90 (1H) ppm.
¹H-NMR (CDCl₃) von B: δ= 0,59 (1H), 0,64 (1H), 0,75 (1H), 0,91 (3H), 0,94 (1H), 1,04 (1H), 1,17 (3H), 1,19-1,74 (9H), 1,96 (1H), 2,07-2,35 (5H), 2,46 (1H), 4,52 (1H), 4,59 (1H), 5,71 (1H), 5,75 (1H), 5,90 (1H) ppm.

### Beispiel 16:

### 7α-Vinyl-15α,16α-methylen-17α-(1'-propenyl)-17β-3'-oxidoestra-4-en-3-on (A) und 7β-Vinyl-15α,16α-methylen-17α-(1'-propenyl)-17β-3'-oxidoestra-4-en-3-on (B)

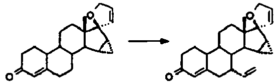

In Analogie zu Beispiel 4 setzt man 250 mg der nach Beispiel 13 dargestellten Verbindung unter Verwendung von Vinylmagnesiumchlorid um und isoliert nach Aufarbeitung und Reinigung 21,4 mg der Titelverbindung A sowie 4,1 mg der Titelverbindung B.
¹H-NMR (CD₂Cl₂) von A: δ= 0,46 (1H), 0,65 (1H), 0,88-1,06 (3H), 1,17 (3H), 1,13-1,30 (2H), 1,44-1,87 (4H), 2,11 (1H), 2,19-2,37 (3H), 2,48 (1H), 2,60 (1H), 2,79 (1H), 4,51 (1H), 4,58 (1H), 5,09 (1H), 5,18 (1H), 5,32 (1H), 5,67-5,77 (2H), 5,79 (1H), 5,88 (1H) ppm.
¹H-NMR (CD₂Cl₂) von B: δ= 0,47 (1H), 0,66 (1H), 0,73-0,81 (2H), 1,06 (1H), 1,15 (3H), 1,17-1,65 (7H), 1,73 (1H), 2,00-2,37 (6H), 4,51 (1H), 4,58 (1H), 4,92 (1H), 5,06 (1H), 5,70 (1H), 5,76 (1H), 5,86-5,99 (2H) ppm.

### Beispiel 17:

### 7α-Cyclopropyl-15α,16α-methylen-17α-(1'-propenyl)-17β-3'-oxidoestra-4-en-3-on (A) und 7β-Cyclopropyl-15α,16α-methylen-17α-(1'-propenyl)-17β-3'-oxidoestra-4-en-3-on (B)

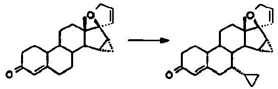

In Analogie zu Beispiel 4 setzt man 250 mg der nach Beispiel 13 dargestellten Verbindung unter Verwendung von Cyclopropylmagnesiumbromid um und isoliert nach Aufarbeitung und Reinigung 47 mg der Titelverbindung A sowie 5,3 mg der Titelverbindung B.
¹H-NMR (CD₂Cl₂) von A: δ= -0,07 (1H), 0,35-0,62 (5H), 0,93-1,30 (8H), 1,17 (3H), 1,48-1,62 (2H), 1,77-1,89 (2H), 2,11 (1H), 2,22-2,38 (3H), 2,44 (1H), 2,53 (1H), 4,51 (1H), 4,60 (1H), 5,77 (1H), 5,82 (1H), 5,91 (1H) ppm.
¹H-NMR (CD₂Cl₂) von B: δ= 0,02 (1H), 0,40-0,73 (7H), 0,94-1,04 (2H), 1,19 (3H), 1,22-1,30 (4H), 1,52-1,77 (4H), 2,08-2,30 (5H), 2,58 (1H), 4,52 (1H), 4,60 (1H), 5,71-5,74 (2H), 5,90 (1H) ppm.

### Beispiel 18

Inerte, intrauterin implantierbare Depotsysteme aus einem biologisch abbaubaren Polymer bzw. einem synthetischen Silikon-Polymer, bestehend aus einem wirkstoffhaltigen Kern in entsprechendem Polymer-Wirkstoff-Mischungsverhältnis, umgeben von einer die gewünschte tägliche Freisetzungsrate gewährleistenden Polymermembran, werden in das Uteruslumen von Ratten verbracht. Die weiblichen Tiere werden vorher kastriert und mit Estradiol über drei Tage vorbehandelt. Die Implantate von unterschiedlicher Länge (5-20 mm) und einem begrenzten Durchmesser (1.1 bis 2 mm) verbleiben zwischen 4 und 14 Tage im Rattenuterus, um die lokale wie sy-stemische gestagene Wirkung des freigesetzten Wirkstoffes anhand verschiedener Parameter in unterschiedlichen Gewebe zu untersuchen. Folgende Parameter werden ermittelt: 1) gestagene lokale Wirkung am Uterus anhand des Uterusgewichts, der histologisch erfassbaren Epithelhöhe und der Expression gestagenregulierter Markergene (z.B. IGFBP-1); 2) gestagene systemische Wirkung an der Mamma anhand der Expression gestagenregulierter Markergene (z.B. RankL), 3) gestagene systemische Wirkung an der Hypophyse anhand des LH-Spiegels (Absenkung des estrogen-induziert erhöhten LH-Spiegels).

Die Verbindungen der vorliegenden Erfindung zeigen einen signifikanten gestagenen Effekt im Uterus der vergleichbar mit einer entsprechenden Behandlung mit einem Levonorgestrel enthaltenden Depotsystems wie MIRENA^{®} ist.

**Tabelle 1: Rezeptorbindungswerte**

| | | Rezeptorbindung | | | | | |
|---|---|---|---|---|---|---|---|
| | | Progesteronrezeptor (PR) | | Mineralocorticoidrezeptor (MR) | Androgenrezeptor | | |
| Bsp. | Struktur | IC50 [nM] | Competition factor | Competition factor | IC50 [nM] | Competition factor | KF PR / KF MR |
| | 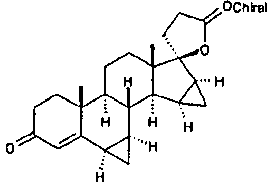 | 43,3 | 2,7 | 0,5 | 630 | 37 | 5,40 |
| 1 | 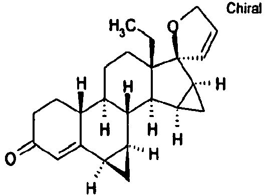 | 27 | 1,04 | 1,8 | 160 | 17,8 | 0,58 |
| 2 | 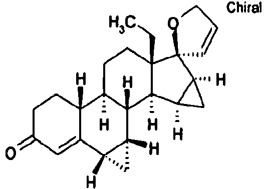 | 470 | 8,69 | 3,6 | 150 | 16,3 | 2,41 |
| 3 | 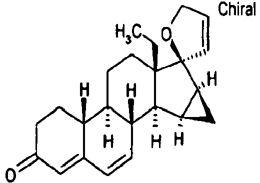 | 53 | 2,43 | 3,4 | 410 | 19,3 | 0,71 |
| 4A | 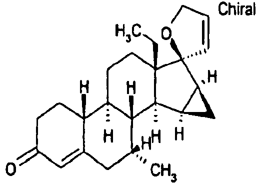 | | 1,89 | 3,8 | 78 | 3,2 | 0,50 |
| 5A | 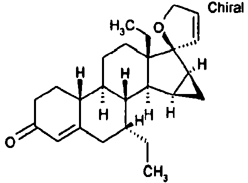 | 140 | 6,15 | 2,3 | 100 | 5,4 | 2,67 |
| 5B | 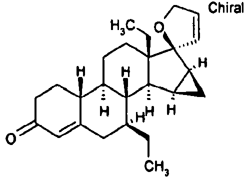 | 130 | 6,03 | 15,0 | 990 | 42,8 | 0,40 |
| 6A | | 44 | 4,30 | 1,8 | 56 | 3,1 | 2,39 |
| 7A | 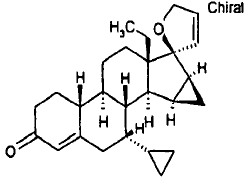 | 120 | 4,42 | 4,4 | 240 | 8,4 | 1,00 |
| 8 | 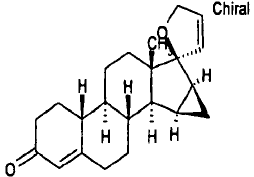 | 24 | 1,03 | 1,8 | 72 | 4,0 | 0,57 |
| 10 | 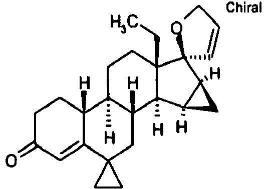 | 33 | 1,08 | 0,8 | 53 | 2,8 | 1,35 |
| 11 | 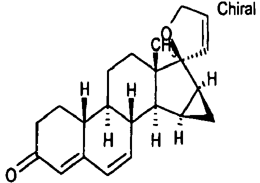 | | | | | | |
| 12 | 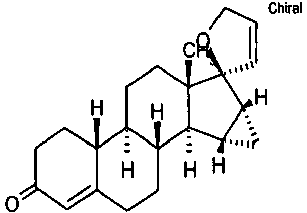 | 29 | 0,79 | 1,0 | 6 | 120,0 | 0,79 |
| 13 | 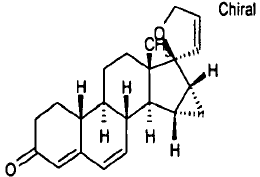 | 56 | 2,04 | 0,9 | 2100 | 74,7 | 2,27 |
| 14A | 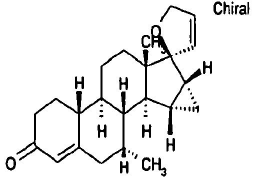 | 44 | 1,60 | 3,1 | 82 | 2,9 | 0,52 |
| 15A | 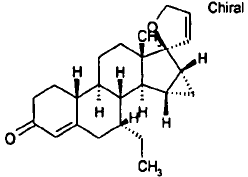 | 56 | 2,31 | 3,7 | 85 | 3,8 | 0,62 |
| 15B | 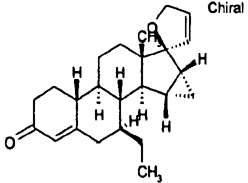 | | | | | | |
| 16A | 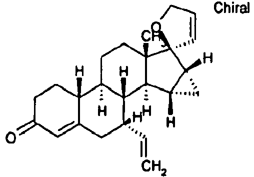 | 40 | 1,66 | 10,5 | 77 | 3,7 | 0,16 |
| 16B | 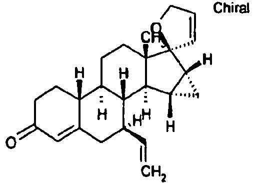 | | | | | | |
| 17A | 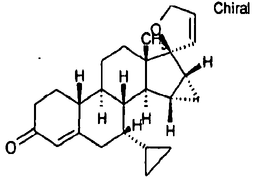 | 13 | 0,59 | 2,5 | 15 | 1,0 | 0,24 |
| 17B | | | | | | | |

**Tabelle 2: Werte zur in vitro-Transaktivierung**

| | | *In vitro*-Transaktivierung | | | | | | | |
|---|---|---|---|---|---|---|---|---|---|
| | | Progesteronrezeptor | | Mineralocorticoidrezeptor | | Androgenrezeptor | | | |
| Bsp. | Struktur | Agonismus EC50 [nM] | Agonismus Wirksam-keit [%] | Antagonismus IC50 [nM] | Antagonismus Wirksamkeit [%] | Agonismus EC50 [nM] | Agonismus Wirksamkeit [%] | Antagonismus IC50 [nM] | Antagonismus Wirksamkeit [%] |
| A | 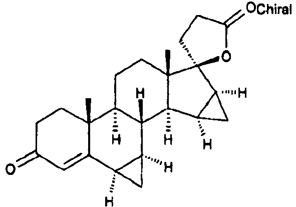 | 88 | 72,2 | 3,3 | 64,1 | 112,5 | 24,26 | 27 | 54,58 |
| 1 | 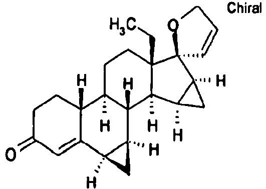 | | | Partial Agonist | | 9 | 68 | 11 | 35,3 |
| 2 | 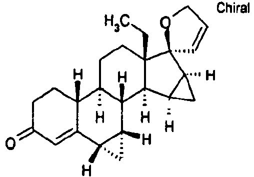 | | | 35 | 118,8 | 4,8 | 51,5 | 41 | 47,8 |
| 3 | 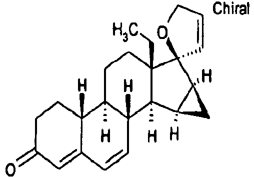 | | | 110 | 71,0 | 39 | 60,32 | 1000 | 5 |
| 4A | 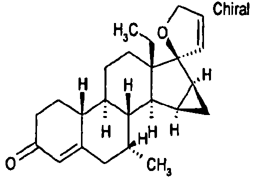 | 0,8 | 23,2 | 9 | 44,5 | 3,2 | 80,97 | 1000 | 5 |
| 5A | 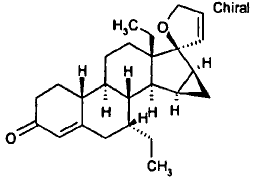 | 13,0 | 20,6 | 140 | 100,2 | 4,6 | 81,2 | 1000 | 5 |
| 5B | 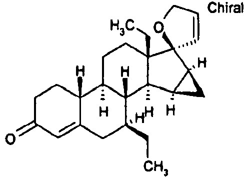 | 36,0 | 23,2 | 130 | 67,7 | 100 | 23,9 | 130 | 63,82 |
| 6A | | 7,8 | 34,6 | 5 | 123,1 | 1,9 | 93,47 | 1000 | 5 |
| 7A | 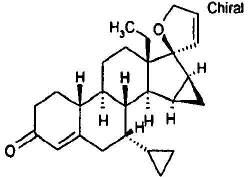 | 7,5 | 37,1 | Partial Agonist | | 17 | 82,3 | 1000 | 5 |
| 8 | 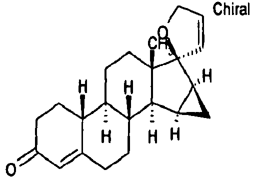 | 1,6 | 77,5 | 95 | 106,2 | 10 | 58,71 | 1000 | 5 |
| 10 | 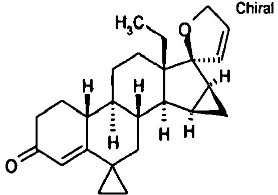 | 0,1 | 45,2 | Partial Agonist | | 1,2 | 106,55 | 1000 | 5 |
| 11 | 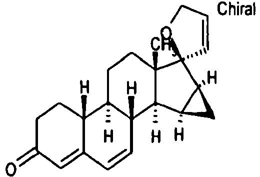 | | | | | | | | |
| 12 | 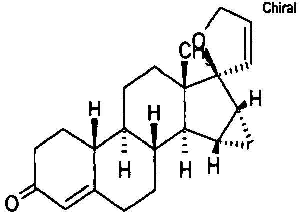 | 0,1 | 71,1 | Agonistisch | | 41 | 62,66 | 1000 | 5 |
| 13 | 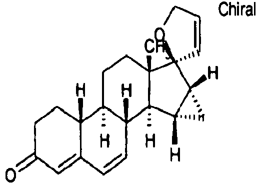 | 8,4 | 62,8 | 46 | 86,1 | 200 | 10,57 | 130 | 81,32 |
| 14A | 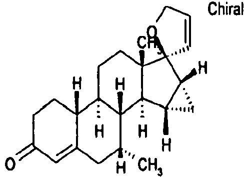 | 0,7 | 96,0 | 72 | 60,9 | 4,6 | 56,06 | 1000 | 5 |
| 15A | 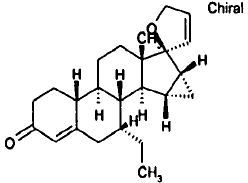 | 0,9 | 52,9 | Agonistisch | | 9,3 | 73,74 | 1000 | 5 |
| 15B | 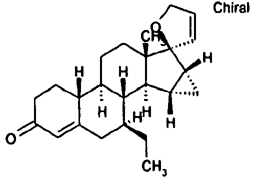 | | | | | | | | |
| 16A | 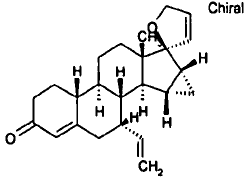 | 1,0 | 74,1 | Partial Agonist | | 4,4 | 72,56 | 1000 | 5 |
| 16B | 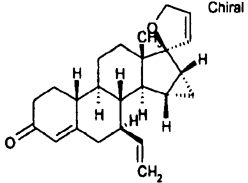 | | | | | | | | |
| 17A | 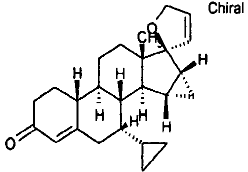 | 4,5 | 82,7 | 110 | 71,5 | 11 | 65,81 | 1000 | 5 |
| 17B | | | | | | | | | |

## Patentansprüche

1. 15,16-Methylen-17-(1'-propenyl)-17-3'-oxidoestra-4-en-3-on-Derivat mit der allgemeinen chemischen Formel I 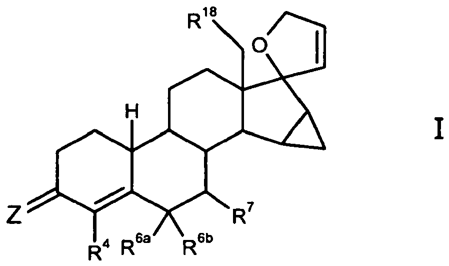 worin
Z ausgewählt ist aus der Gruppe, umfassend Sauerstoff, zwei Wasserstoffatome, NOR' und NNHSO₂R', worin R' Wasserstoff, C₁-C₁₀Alkyl, Aryl oder C₇-C₂₀-Aralkyl ist,
R⁴ ausgewählt ist aus der Gruppe, umfassend Wasserstoff, Fluor, Chlor oder Brom
,
ferner entweder:
R^{6a}, R^{6b} jeweils unabhängig voneinander ausgewählt sind aus der Gruppe, umfassend Wasserstoff, C₁-C₁₀-Alkyl, C₂-C₁₀-Alkenyl und C₂-C₁₀-Alkinyl, oder gemeinsam Methylen oder 1,2-Ethandiyl bilden und
R⁷ ausgewählt ist aus der Gruppe, umfassend Wasserstoff, C₁-C₁₀-Alkyl, C₃-C₆-Cycloalkyl, C₂-C₁₀-Alkenyl und C₂-C₁₀-Alkinyl,
oder:
R^{6a}, R⁷ gemeinsam Sauerstoff oder eine Methylengruppe bilden oder unter Bildung einer Doppelbindung zwischen C⁶ und C⁷ entfallen und
R^{6b} ausgewählt ist aus der Gruppe, umfassend Wasserstoff, C₁-C₁₀-Alkyl, C₂-C₁₀-Alkenyl und C₂-C₁₀-Alkinyl und
R¹⁸ ausgewählt ist aus der Gruppe, umfassend Wasserstoff und C₁-C₃-Alkyl,
sowie dessen Solvate, Hydrate und Salze und einschließlich aller Kristallmodifikationen und aller Stereoisomere.

2. 15,16-Methylen-17-(1'-propenyl)-17-3'-oxidoestra-4-en-3-on-Derivat nach Anspruch 1, **dadurch gekennzeichnet, dass** Z ausgewählt ist aus der Gruppe, umfassend Sauerstoff, NOR' und NNHSO₂R'.

3. 15,16-Methylen-17-(1'-propenyl)-17-3'-oxidoestra-4-en-3-on-Derivat nach einem der vorstehenden Ansprüche, **dadurch gekennzeichnet, dass** Z für Sauerstoff steht.

4. 15,16-Methylen-17-(1'-propenyl)-17-3'-oxidoestra-4-en-3-on-Derivat nach einem der vorstehenden Ansprüche, **dadurch gekennzeichnet, dass** R⁴ ausgewählt ist aus der Gruppe, umfassend Wasserstoff und Chlor.

5. 15,16-Methylen-17-(1'-propenyl)-17-3'-oxidoestra-4-en-3-on-Derivat nach einem der vorstehenden Ansprüche, **dadurch gekennzeichnet, dass** R^{6a} und R^{6b} gemeinsam 1,2-Ethandiyl bilden oder jeweils Wasserstoff sind.

6. 15,16-Methylen-17-(1'-propenyl)-17-3'-oxidoestra-4-en-3-on-Derivat nach einem der vorstehenden Ansprüche, **dadurch gekennzeichnet, dass** R⁷ ausgewählt ist aus der Gruppe, umfassend Wasserstoff, Methyl, Ethyl und Vinyl.

7. 15,16-Methylen-17-(1'-propenyl)-17-3'-oxidoestra-4-en-3-on-Derivat nach einem der Ansprüche 1-4, **dadurch gekennzeichnet, dass** R^{6a} und R⁷ gemeinsam eine Methylengruppe bilden.

8. 15,16-Methylen-17-(1'-propenyl)-17-3'-oxidoestra-4-en-3-on-Derivat nach einem der Ansprüche 1 - 4, **dadurch gekennzeichnet, dass** R^{6a} und R⁷ unter Bildung einer Doppelbindung zwischen C⁶ und C⁷ entfallen.

9. 15,16-Methylen-17-(1'-propenyl)-17-3'-oxidoestra-4-en-3-on-Derivat nach einem der vorstehenden Ansprüche, **dadurch gekennzeichnet, dass** R¹⁸ ausgewählt ist aus der Gruppe, umfassend Wasserstoff und Methyl.

10. 15,16-Methylen-17-(1'-propenyl)-17-3'-oxidoestra-4-en-3-on-Derivat nach einem der vorstehenden Ansprüche, ausgewählt aus der Gruppe, umfassend
• 17α-(1'-Propenyl)-15α,16α-methylen-17β-3'-oxidoestra-4-en-3-on
• 17α-(1'-Propenyl)-15β,16β-methylen-17β-3'-oxidoestra-4-en-3-on
• 7α-Methyl-15α,16α-methylen-17α-(1'-propenyl)-17β-3'-oxidoestra-4-en-3-on
• 7β-Methyl-15α,16α-methylen-17α-(1'-propenyl)-17β-3'-oxidoestra-4-en-3-on
• 7α-Methyl-15β,16β-methylen-17α-(1'-propenyl)-17β-3'-oxidoestra-4-en-3-on
• 7β-Methyl-15β,16β-methylen-17α-(1'-propenyl)-17β-3'-oxidoestra-4-en-3-on
• 7α-Ethyl-15α,16α-methylen-17α-(1'-propenyl)-17β-3'-oxidoestra-4-en-3-on
• 7β-Ethyl-15α, 16α-methylen-17α-(1'-propenyl)-17β-3'-oxidoestra-4-en-3-on
• 7α-Ethyl-15β,16β-methylen-17α-(1'-propenyl)-17β-3'-oxidoestra-4-en-3-on
• 7β-Ethyl-15β,16β-methylen-17α-(1'-propenyl)-17β-3'-oxidoestra-4-en-3-on
• 7α-Vinyl-15α,16α-methylen-17α-(1'-propenyl)-17β-3'-oxidoestra-4-en-3-on
• 7β-Vinyl-15α,16α-methylen-17α-(1'-propenyl)-17β-3'-oxidoestra-4-en-3-on
• 7α-Vinyl-15β,16β-methylen-17α-(1'-propenyl)-17β-3'-oxidoestra-4-en-3-on
• 7β-Vinyl-15β,16β-methylen-17α-(1'-propenyl)-17β-3'-oxidoestra-4-en-3-on
• 7α-Cyclopropyl-15α,16α-methylen-17α-(1'-propenyl)-17β-3'-oxidoestra-4-en-3-on
• 7β-Cyclopropyl-15a,16a-methylen-17a-(1'-propenyl)-17β-3'-oxidoestra-4-en-3-on
• 7α-Cyclopropyl-15β,16β-methylen-17α-(1'-propenyl)-17β-3'-oxidoestra-4-en-3-on
• 7β-Cyclopropyl-15β,16β-methylen-17α-(1'-propenyl)-17β-3'-oxidoestra-4-en-3-on
• 6-Methylen-15α,16α-methylen-17α-(1'-propenyl)-17β-3'-oxidoestra-4-en-3-on
• 6-Methylen-15β,16β-methylen-17α-(1'-propenyl)-17β-3'-oxidoestra-4-en-3-on
• 6,6-(1,2-Ethandiyl)-15α,16α-methylen-17α-(1'-propenyl)-17β-3'-oxidoestra-4-en-3-on
• 6,6-(1,2-Ethandiyl)-15β,16β-methylen-17α-(1'-propenyl)-17β-3'-oxidoestra-4-en-3-on
• 6α,7α;15α,16α-Bismethylen-17α-(1'-propenyl)-17β-3'-oxidoestra-4-en-3-on
• 6β,7β;15α,16α-Bismethylen-17α-(1'-propenyl)-17β-3'-oxidoestra-4-en-3-on
• 6α,7α;15β,16β-Bismethylen-17α-(1'-propenyl)-17β-3'-oxidoestra-4-en-3-on
• 6β,7β;15β,16β-Bismethylen-17α-(1'-propenyl)-17β-3'-oxidoestra-4-en-3-on
• 17α-(1'-Propenyl)-15α,16α-methylen-17β-3'-oxidoestra-4,6-dien-3-on
• 17α-(1'-Propenyl)-15β,16β-methylen-17β-3'-oxidoestra-4,6-dien-3-on
• (E/Z)-3-(Hydroxyimino)-17α-(1'-propenyl)-15α,16α-methylen-17β-3'-oxidoestra-4-en
• (E/Z)-3-(Hydroxyimino)-17α-(1'-propenyl)-15β,16β-methylen-17β-3'-oxidoestra-4-en
• (E/Z)-3-(Hydroxyimino)-7α-methyl-15α,16α-methylen-17α-(1'-propenyl)-17β-3'-oxidoestra-4-en
• (E/Z)-3-(Hydroxyimino)-7β-methyl-15α,16α-methylen-17α-(1'-propenyl)-17β-3'-oxidoestra-4-en
• (E/Z)-3-(Hydroxyimino)-7α-methyl-15β,16β-methylen-17α-(1'-propenyl)-17β-3'-oxidoestra-4-en
• (E/Z)-3-(Hydroxyimino)-7β-methyl-15β,16β-methylen-17α-(1'-propenyl)-17β-3'-oxidoestra-4-en
• (E/Z)-3-(Hydroxyimino)-7α-ethyl-15α,16α-methylen-17α-(1'-propenyl)-17β-3'-oxidoestra-4-en
• (E/Z)-3-(Hydroxyimino)-7β-ethyl-15α,16α-methylen-17α-(1'-propenyl)-17β-3'-oxidoestra-4-en
• (E/Z)-3-(Hydroxyimino)-7α-ethyl-15β,16β-methylen-17α-(1'-propenyl)-17β-3'-oxidoestra-4-en
• (E/Z)-3-(Hydroxyimino)-7β-ethyl-15β,16β-methylen-17α-(1'-propenyl)-17β-3'-oxidoestra-4-en
• (E/Z)-3-(Hydroxyimino)-7α-vinyl-15α,16α-methylen-17α-(1'-propenyl)-17β-3'-oxidoestra-4-en
• (E/Z)-3-(Hydroxyimino)-7β-vinyl-15α,16α-methylen-17α-(1'-propenyl)-17β-3'-oxidoestra-4-en
• (E/Z)-3-(Hydroxyimino)-7α-vinyl-15β,16β-methylen-17α-(1'-propenyl)-17β-3'-oxidoestra-4-en
• (E/Z)-3-(Hydroxyimino)-7β-vinyl-15β,16β-methylen-17α-(1'-propenyl)-17β-3'-oxidoestra-4-en
• (E/Z)-3-(Hydroxyimino)-7α-cyclopropyl-15α,16α-methylen-17α-(1'-propenyl)-17β-3'-oxidoestra-4-en
• (E/Z)-3-(Hydroxyimino)-7β-cyclopropyl-15α,16α-methylen-17α-(1'-propenyl)-17β-3'-oxidoestra-4-en
• (E/Z)-3-(Hydroxyimino)-7α-cyclopropyl-15β,16β-methylen-17α-(1'-propenyl)-17β-3'-oxidoestra-4-en
• (E/Z)-3-(Hydroxyimino)-7β-cyclopropyl-15β,16β-methylen-17α-(1'-propenyl)-17β-3'-oxidoestra-4-en
• (E/Z)-3-(Hydroxyimino)-6-methylen-17α-(1'-propenyl)-15α,16α-methylen-17β-3'-oxidoestra-4-en
• (E/Z)-3-(Hydroxyimino)-6-methylen-17α-(1'-propenyl)-15β,16β-methylen-17β-3'-oxidoestra-4-en
• (E/Z)-3-(Hydroxyimino)-6,6-(1,2-ethandiyl)-17α-(1'-propenyl)-15α,16α-methylen-17β-3'-oxidoestra-4-en
• (E/Z)-3-(Hydroxyimino)-6,6-(1,2-ethandiyl)-17α-(1'-propenyl)-15β,16β-methylen-17β-3'-oxidoestra-4-en
• (E/Z)-3-(Hydroxyimino)-6α,7α;15α,16α-bismethylen-17α-(1'-propenyl)-17β-3'-oxidoestra-4-en
• (E/Z)-3-(Hydroxyimino)-6β,7β;15α,16α-bismethylen-17α-(1'-propenyl)-17β-3'-oxidoestra-4-en
• (E/Z)-3-(Hydroxyimino)-6α,7α;15β,16β-bismethylen-17α-(1'-propenyl)-17β-3'-oxidoestra-4-en
• (E/Z)-3-(Hydroxyimino)-6β,7β;15β,16β-bismethylen-17α-(1'-propenyl)-17β-3'-oxidoestra-4-en
• (E/Z)-3-(Hydroxyimino)-17α-(1'-propenyl)-15α,16α-methylen-17β-3'-oxidoestra-4,6-dien
• (E/Z)-3-(Hydroxyimino)-17α-(1'-propenyl)-15β,16β-methylen-17β-3'-oxidoestra-4,6-dien
• 17α-(1'-Propenyl)-18-methyl-15α,16α-methylen-17β-3'-oxidoestra-4-en-3-on
• 17α-(1'-Propenyl)-18-methyl-15β,16β-methylen-17β-3'-oxidoestra-4-en-3-on
• 7α,18-Dimethyl-15α,16α-methylen-17α-(1'-propenyl)-17β-3'-oxidoestra-4-en-3-on
• 7β,18-Dimethyl-15α,16α-methylen-17α-(1'-propenyl)-17β-3'-oxidoestra-4-en-3-on
• 7α,18-Dimethyl-15β,16β-methylen-17α-(1'-propenyl)-17β-3'-oxidoestra-4-en-3-on
• 7β,18-Dimethyl-15β,16β-methylen-17α-(1'-propenyl)-17β-3'-oxidoestra-4-en-3-on
• 7α-Ethyl-18-methyl-15a,16α-methylen-17α-(1'-propenyl)-17β-3'-oxidoestra-4-en-3-on
• 7β-Ethyl-18-methyl-15α,16α-methylen-17α-(1'-propenyl)-17β-3'-oxidoestra-4-en-3-on
• 7α-Ethyl-18-methyl-15β,16β-methylen-17α-(1'-propenyl)-17β-3'-oxidoestra-4-en-3-on
• 7β-Ethyl-18-methyl-15β,16β-methylen-17α-(1'-propenyl)-17β-3'-oxidoestra-4-en-3-on
• 7α-Vinyl-18-methyl-15α,16α-methylen-17α-(1'-propenyl)-17β-3'-oxidoestra-4-en-3-on
• 7β-Vinyl-18-methyl-15α,16α-methylen-17α-(1'-propenyl)-17β-3'-oxidoestra-4-en-3-on
• 7α-Vinyl-18-methyl-15β,16β-methylen-17α-(1'-propenyl)-17β-3'-oxidoestra-4-en-3-on
• 7β-Vinyl-18-methyl-15β,16β-methylen-17α-(1'-propenyl)-17β-3'-oxidoestra-4-en-3-on
• 7α-Cyclopropyl-18-methyl-15α,16α-methylen-17α-(1'-propenyl)-17β-3'-oxidoestra-4-en-3-on
• 7β-Cyclopropyl-18-methyl-15α,16α-methylen-17α-(1'-propenyl)-17β-3'-oxidoestra-4-en-3-on
• 7α-Cyclopropyl-18-methyl-15β,16β-methylen-17α-(1'-propenyl)-17β-3'-oxidoestra-4-en-3-on
• 7β-Cyclopropyl-18-methyl-15β,16β-methylen-17α-(1'-propenyl)-17β-3'-oxidoestra-4-en-3-on
• 6-Methylen-18-methyl-15α,16α-methylen-17α-(1'-propenyl)-17β-3'-oxidoestra-4-en-3-on
• 6-Methylen-18-methyl-15β,16β-methylen-17α-(1'-propenyl)-17β-3'-oxidoestra-4-en-3-on
• 6,6-(1,2-Ethandiyl)-18-methyl-15α,16α-methylen-17α-(1'-propenyl)-17β-3'-oxidoestra-4-en-3-on
• 6,6-(1,2-Ethandiyl)-18-methyl-15β,16β-methylen-17α-(1'-propenyl)-17β-3'-oxidoestra-4-en-3-on
• 6α,7α;15α,16α-Bismethylen-18-methyl-17α-(1'-propenyl)-17β-3'-oxidoestra-4-en-3-on
• 6β,7β;15α,16α-Bismethylen-18-methyl-17α-(1'-propenyl)-17β-3'-oxidoestra-4-en-3-on
• 6α,7α;15β,16β-Bismethylen-18-methyl-17α-(1'-propenyl)-17β-3'-oxidoestra-4-en-3-on
• 6β,7β;15β,16β-Bismethylen-18-methyl-17α-(1'-propenyl)-17β-3'-oxidoestra-4-en-3-on
• 17α-(1'-Propenyl)-18-methyl-15α,16α-methylen-17β-3'-oxidoestra-4,6-dien-3-on
• 17α-(1'-Propenyl)-18-methyl-15β,16β-methylen-17β-3'-oxidoestra-4,6-dien-3-on
• (E/Z)-3-(Hydroxyimino)-18-methyl-17α-(1'-propenyl)-15α,16α-methylen-17β-3'-oxidoestra-4-en
• (E/Z)-3-(Hydroxyimino)-18-methyl-17α-(1'-propenyl)-15β,16β-methylen-17β-3'-oxidoestra-4-en
• (E/Z)-3-(Hydroxyimino)-7α,18-bismethyl-15α,16α-methylen-17α-(1'-propenyl)-17β-3'-oxidoestra-4-en
• (E/Z)-3-(Hydroxyimino)-7β,18-bismethyl-15α,16α-methylen-17α-(1'-propenyl)-17β-3'-oxidoestra-4-en
• (E/Z)-3-(Hydroxyimino)-7α,18-bismethyl-15β,16β-methylen-17α-(1'-propenyl)-17β-3'-oxidoestra-4-en
• (E/Z)-3-(Hydroxyimino)-7β,18-bismethyl-15β,16β-methylen-17α-(1'-propenyl)-17β-3'-oxidoestra-4-en
• (E/Z)-3-(Hydroxyimino)-7α-ethyl-18-methyl-15α,16α-methylen-17α-(1'-propenyl)-17β-3'-oxidoestra-4-en
• (E/Z)-3-(Hydroxyimino)-7β-ethyl-18-methyl-15α,16α-methylen-17α-(1'-propenyl)-17β-3'-oxidoestra-4-en
• (E/Z)-3-(Hydroxyimino)-7α-ethyl-18-methyl-15β,16β-methylen-17α-(1'-propenyl)-17β-3'-oxidoestra-4-en
• (E/Z)-3-(Hydroxyimino)-7β-ethyl-18-methyl-15β,16β-methylen-17α-(1'-propenyl)-17β-3'-oxidoestra-4-en
• (E/Z)-3-(Hydroxyimino)-7α-vinyl-18-methyl-15α,16α-methylen-17α-(1'-propenyl)-17β-3'-oxidoestra-4-en
• (E/Z)-3-(Hydroxyimino)-7β-vinyl-18-methyl-15α,16α-methylen-17α-(1'-propenyl)-17β-3'-oxidoestra-4-en
• (E/Z)-3-(Hydroxyimino)-7α-vinyl-18-methyl-15β,16β-methylen-17α-(1'-propenyl)-17β-3'-oxidoestra-4-en
• (E/Z)-3-(Hydroxyimino)-7β-vinyl-18-methyl-15β,16β-methylen-17α-(1'-propenyl)-17β-3'-oxidoestra-4-en
• (E/Z)-3-(Hydroxyimino)-7α-cyclopropyl-18-methyl-15α,16α-methylen-17α-(1'-propenyl)-17β-3'-oxidoestra-4-en
• (E/Z)-3-(Hydroxyimino)-7β-cyclopropyl-18-methyl-15α,16α-methylen-17a-(1'-propenyl)-17β-3'-oxidoestra-4-en
• (E/Z)-3-(Hydroxyimino)-7α-cyclopropyl-18-methyl-15β,16β-methylen-17α-(1'-propenyl)-17β-3'-oxidoestra-4-en
• (E/Z)-3-(Hydroxyimino)-7β-cyclopropyl-18-methyl-15β,16β-methylen-17α-(1'-propenyl)-17β-3'-oxidoestra-4-en
• (E/Z)-3-(Hydroxyimino)-18-methyl-6-methylen-17α-(1'-propenyl)-15α,16α-methylen-17β-3'-oxidoestra-4-en
• (E/Z)-3-(Hydroxyimino)-18-methyl-6-methylen-17α-(1'-propenyl)-15β,16β-methylen-17β-3'-oxidoestra-4-en
• (E/Z)-3-(Hydroxyimino)-6,6-(1,2-ethandiyl)-17α-(1'-propenyl)-18-methyl-15α,16a-methylen-17β-3'-oxidoestra-4-en
• (E/Z)-3-(Hydroxyimino)-6,6-(1,2-ethandiyl)-17α-(1'-propenyl)-18-methyl-15β,16β-methylen-17β-3'-oxidoestra-4-en
• (E/Z)-3-(Hydroxyimino)-6α,7α;15α,16α-bismethylen-18-methyl-17α-(1'-propenyl)-17β-3'-oxidoestra-4-en
• (E/Z)-3-(Hydroxyimino)-6β,7β;15α,16α-bismethylen-18-methyl-17α-(1'-propenyl)-17β-3'-oxidoestra-4-en
• (E/Z)-3-(Hydroxyimino)-6α,7α;15β,16β-bismethylen-18-methyl-17α-(1'-propenyl)-17β-3'-oxidoestra-4-en
• (E/Z)-3-(Hydroxyimino)-6β,7β;15β,16β-bismethylen-18-methyl-17α-(1'-propenyl)-17β-3'-oxidoestra-4-en
• (E/Z)-3-(Hydroxyimino)-17α-(1'-propenyl)-18-methyl-15α,16α-methylen-17β-3'-oxidoestra-4,6-dien
• (E/Z)-3-(Hydroxyimino)-17α-(1'-propenyl)-18-methyl-15β,16β-methylen-17β-3'-oxidoestra-4,6-dien

11. 15,16-Methylen-17-(1'-propenyl)-17-3'-oxidoestra-4-en-3-on-Derivat nach einem der vorstehenden Ansprüche zur oralen Kontrazeption und zur Behandlung von prä-, peri- und postmenopausalen Beschwerden.

12. Verwendung des 15,16-Methylen-17-(1'-propenyl)-17-3'-oxidoestra-4-en-3-on-Derivats nach einem der Ansprüche 1-11 zur Herstellung eines Arzneimittels zur oralen Kontrazeption und zur Behandlung von prä-, peri- und postmenopausalen Beschwerden.

13. Verwendung nach Anspruch 12, **dadurch gekennzeichnet, dass** das Arzneimittel gestagene, antimineralcorticoide und neutrale bis leicht androgene Wirkung aufweist.

14. Arzneimittel, enthaltend mindestens ein 15,16-Methylen-17-(1'-propenyl)-17-3'-oxidoestra-4-en-3-on-Derivat nach einem der Ansprüche 1-11 sowie mindestens einen geeigneten pharmazeutisch unbedenklichen Zusatzstoff.

15. Arzneimittel nach Anspruch 14, enthaltend außerdem mindestens ein Estrogen.

16. Arzneimittel nach Anspruch 15, **dadurch gekennzeichnet, dass** das Estrogen Ethinylestradiol ist.

17. Arzneimittel nach Anspruch 15, **dadurch gekennzeichnet, dass** das Estrogen Estradiolvalerat ist.

18. Arzneimittel nach Anspruch 15, **dadurch gekennzeichnet, dass** das Estrogen ein natürliches Estrogen ist.

19. Arzneimittel nach Anspruch 18, **dadurch gekennzeichnet, dass** das natürliche Estrogen Estradiol ist.

20. Arzneimittel nach Anspruch 18, **dadurch gekennzeichnet, dass** das natürliche Estrogen ein konjugiertes Estrogen ist.

21. Verwendung des 15,16-Methylen-17-(1'-propenyl)-17-3'-oxidoestra-4-en-3-on-Derivats nach einem der Ansprüche 1 - 9 zur Herstellung eines Arzneimittels zur intrauterinen Anwendung.

22. Verwendung nach Anspruch 21 zur Herstellung eines intrauterinen Systems (I-US).

23. Arzneimittel enthaltend mindestens ein 15,16-Methylen-17-(1'-propenyl)-17-3'-oxidoestra-4-en-3-on-Derivat nach einem der Ansprüche 1-9 sowie mindestens einen geeigneten pharmazeutisch unbedenklichen Trägerstoff, **dadurch gekennzeichnet, dass** es zur intrauterinen Anwendung hergerichtet ist.

24. Arzneimittel nach Anspruch 23, **dadurch gekennzeichnet, dass** es ein intrauterines System ist.

## Claims

1. 15,16-Methylene-17-(1'-propenyl)-17-3'-oxidoestra-4-en-3-one derivative with the general chemical formula I 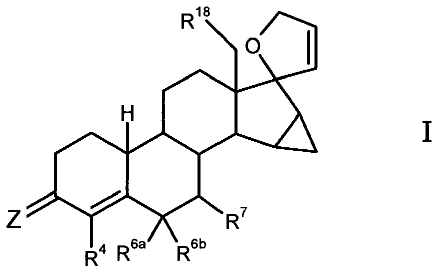 in which
Z is selected from the group comprising oxygen, two hydrogen atoms, NOR' and NNHSO₂R', where R' is hydrogen, C₁-C₁₀ alkyl, aryl or C₇-C₂₀ aralkyl, and
R⁴ is selected from the group comprising hydrogen, fluorine, chlorine or bromine,
and further, either:
R^{6a}, R^{6b} are each mutually independently selected from the group comprising hydrogen, C₁-C₁₀ alkyl, C₂-C₁₀ alkenyl and C₂-C₁₀ alkynyl, or together form methylene or 1,2-ethanediyl and
R⁷ is selected from the group comprising hydrogen, C₁-C₁₀ alkyl, C₃-C₆-cycloalkyl, C₂-C₁₀ alkenyl and C₂-C₁₀ alkynyl,
or:
R^{6a}, R⁷ together comprise oxygen or a methylene group or are omitted, with the formation of a double bond between C⁶ and C⁷ and
R^{6b} is selected from the group comprising hydrogen, C₁-C₁₀ alkyl, C₂-C₁₀ alkenyl and C₂-C₁₀ alkynyl and
R¹⁸ is selected from the group comprising hydrogen and C₁-C₃ alkyl,
and solvates, hydrates and salts thereof and including all crystal modifications and all stereoisomers.

2. 15,16-Methylene-17-(1'-propenyl)-17-3'-oxidoestra-4-en-3-one derivative according to Claim 1, **characterized in that** Z is selected from the group comprising oxygen, NOR' and NNHSO₂R'.

3. 15,16-Methylene-17-(1'-propenyl)-17-3'-oxidoestra-4-en-3-one derivative according to one of the previous claims, **characterized in that** Z stands for oxygen.

4. 15,16-Methylene-17-(1'-propenyl)-17-3'-oxidoestra-4-en-3-one derivative according to one of the previous claims, **characterized in that** R⁴ is selected from the group comprising hydrogen and chlorine.

5. 15,16-Methylene-17-(1'-propenyl)-17-3'-oxidoestra-4-en-3-one derivative according to one of the previous claims, **characterized in that** R^{6a} and R^{6b} together form 1,2-ethanediyl or are each hydrogen.

6. 15,16-Methylene-17-(1'-propenyl)-17-3'-oxidoestra-4-en-3-one derivative according to one of the previous claims, **characterized in that** R⁷ is selected from the group comprising hydrogen, methyl, ethyl and vinyl.

7. 15,16-Methylene-17-(1'-propenyl)-17-3'-oxidoestra-4-en-3-one derivative according to one of Claims 1-4, **characterized in that** R^{6a} and R⁷ together form a methylene group.

8. 15,16-Methylene-17-(1'-propenyl)-17-3'-oxidoestra-4-en-3-one derivative according to one of Claims 1-4, **characterized in that** R^{6a} and R⁷ are omitted, with the formation of a double bond between C⁶ and C⁷.

9. 15,16-Methylene-17-(1'-propenyl)-17-3'-oxidoestra-4-en-3-one derivative according to one of the previous claims, **characterized in that** R¹⁸ is selected from the group comprising hydrogen and methyl.

10. 15,16-Methylene-17-(1'-propenyl)-17-3'-oxidoestra-4-en-3-one derivative according to one of the previous claims, selected from the group comprising
• 17α-(1'-propenyl)-15α,16α-methylene-17β-3'-oxidoestra-4-en-3 -one
• 17α-(1'-propenyl)-15β,16β-methylene-17β-3'-oxidoestra-4-en-3-one
• 7α-methyl-15α,16α-methylene-17α-(1'-propenyl)-17β-3'-oxidoestra-4-en-3-one
• 7β-methyl-15α,16α-methylene-17α-(1'-propenyl)-17β-3'-oxidoestra-4-en-3-one
• 7α-methyl-15β,16β-methylene-17α-(1'-propenyl)-17β-3'-oxidoestra-4-en-3-one
• 7β-methyl-15β,16β-methylene-17α-(1'-propenyl)-17β-3'-oxidoestra-4-en-3-one
• 7α-ethyl-15α,16α-methylene-17α-(1'-propenyl)-17β-3'-oxidoestra-4-en-3-one
• 7β-ethyl-15α,16α-methylene-17α-(1'-propenyl)-17β-3'-oxidoestra-4-en-3-one
• 7α-ethyl-15β,16β-methylene-17α-(1'-propenyl)-17β-3'-oxidoestra-4-en-3-one
• 7β-ethyl-15β,16β-methylene-17α-(1'-propenyl)-17β-3'-oxidoestra-4-en-3-one
• 7α-vinyl-15α,16α-methylene-17α-(1'-propenyl)-17β-3'-oxidoestra-4-en-3-one
• 7β-vinyl-15α,16α-methylene-17α-(1'-propenyl)-17β-3'-oxidoestra-4-en-3-one
• 7α-vinyl-15β,16β-methylene-17α-(1'-propenyl)-17β-3'-oxidoestra-4-en-3-one
• 7β-vinyl-15β,16β-methylene-17α-(1'-propenyl)-17β-3'-oxidoestra-4-en-3-one
• 7α-cyclopropyl-15α,16α-methylene-17α-(1'-propenyl)-17β-3'-oxidoestra-4-en-3-one
• 7β-cyclopropyl-15α,16α-methylene-17α-(1'-propenyl)-17β-3'-oxidoestra-4-en-3-one
• 7α-cyclopropyl-15β,16β-methylene-17α-(1'-propenyl)-17β-3'-oxidoestra-4-en-3-one
• 7β-cyclopropyl-15β,16β-methylene-17α-(1'-propenyl)-17β-3'-oxidoestra-4-en-3-one
• 6-methylene-15a,16α-methylene-17α-(1'-propenyl)-17β-3'-oxidoestra-4-en-3-one
• 6-methylene-15β,16β-methylene-17α-(1'-propenyl)-17β-3'-oxidoestra-4-en-3-one
• 6,6-(1,2-ethanediyl)-15α,16α-methylene-17α-(1'-propenyl)-17β-3'-oxidoestra-4-en-3-one
• 6,6-(1,2-ethanediyl)-15β,16β-methylene-17α-(1'-propenyl)-17β-3'-oxidoestra-4-en-3-one
• 6α,7α;15α,16α-bismethylene-17α-(1'-propenyl)-17β-3'-oxidoestra-4-en-3-one
• 6β,7β;15α,16α-bismethylene-17α(1'-propenyl)-17β-3'-oxidoestra-4-en-3-one
• 6α,7α;15β,16β-bismethylene-17α-(1'-propenyl)-17β-3'-oxidoestra-4-en-3-one
• 6β,7β;15β,16β-bismethylene-17α-(1'-propenyl)-17β-3'-oxidoestra-4-en-3-one
• 17α-(1'-propenyl)-15α,16α-methylene-17β-3'-oxidoestra-4,6-dien-3-one
• 17α-(1'-propenyl)-15β,16β-methylene-17β-3'-oxidoestra-4,6-dien-3-one
• (E/Z)-3-(hydroxyimino)-17α-(1'-propenyl)-15α,16α-methylene-17β-3'-oxido-estra-4-ene
• (E/Z)-3-(hydroxyimino)-17α-(1'-propenyl)-15β,16β-methylene-17β-3'-oxido-estra-4-ene
• (E/Z)-3-(hydroxyimino)-7α-methyl-15α, 16α-methylene-17α-(1'-propenyl)-17β-3'-oxidoestra-4-ene
• (E/Z)-3-(hydroxyimino)-7β-methyl-15α,16α-methylene-17α-(1'-propenyl)-17β-3'-oxidoestra-4-ene
• (E/Z)-3-(hydroxyimino)-7a-methyl-15β,16β-methylene-17α-(1'-propenyl)-17β-3'-oxidoestra-4-ene
• (E/Z)-3-(hydroxyimino)-7β-methyl-5β, 16β-methylene-17α-(1'-propenyl)-17β-3'-oxidoestra-4-ene
• (E/Z)-3-(hydroxyimino)-7α-ethyl-15α, 16α-methylene-17α-(1'-propenyl)-17β-3'-oxidoestra-4-ene
• (E/Z)-3-(hydroxyimino)-7β-ethyl-15α, 16α-methylene-17α-(1'-propenyl)-17β-3'-oxidoestra-4-ene
• (E/Z)-3-(hydroxyimino)-7α-ethyl-15β,16β-methylene-17α-(1'-propenyl)-17β-3'-oxidoestra-4-ene
• (E/Z)-3-(hydroxyimino)-7β-ethyl-15β,16β-methylene-17α-(1'-propenyl)-17β-3'-oxidoestra-4-ene
• (E/Z)-3-(hydroxyimino)-7α-vinyl-15α, 16α-methylene-17α-(1'-propenyl)-17β-3'-oxidoestra-4-ene
• (E/Z)-3-(hydroxyimino)-7p-vinyl-15a, 16α-methylene-17α-(1'-propenyl)-17β-3'-oxidoestra-4-ene
• (E/Z)-3-(hydroxyimino)-7α-vinyl-15β,16β-methylene-17α-(1'-propenyl)-17β-3'-oxidoestra-4-ene
• (E/Z)-3-(hydroxyimino)-7β-vinyl-15β,16β-methylene-17α-(1'-propenyl)-17β-3'-oxidoestra-4-ene
• (E/Z)-3-(hydroxyimino)-7α-cyclopropyl-15α, 16α-methylene-17α-(1'-propenyl)-17β-3'-oxidoestra-4-ene
• (E/Z)-3-(hydroxyimino)-7β-cyclopropyl-15α,16α-methylene-17α-(1'-propenyl)-17β-3'-oxidoestra-4-ene
• (E/Z)-3-(hydroxyimino)-7α-cyclopropyl-15β,16β-methylene-17α-(1'-propenyl)-17β-3'-oxidoestra-4-ene
• (E/Z)-3-(hydroxyimino)-7β-cyclopropyl-15β,16β-methylene-17α-(1'-propenyl)-17β-3'-oxidoestra-4-ene
• (E/Z)-3-(hydroxyimino)-6-methylene-17α-(1'-propenyl)-15α,16α-methylene-17β-3'-oxidoestra-4-ene
• (E/Z)-3-(hydroxyimino)-6-methylene-17α-(1'-propenyl)-15β,16β-methylene-17β-3'-oxidoestra-4-ene
• (E/Z)-3-(hydroxyimino)-6,6-(1,2-ethandiyl)-17α-(1'-propenyl)-15α,16α-methylene-17β-3'-oxidoestra-4-ene
• (E/Z)-3-(hydroxyimino)-6,6-(1,2-ethandiyl)-17α-(1'-propenyl)-15β,16β-methylene-17β-3'-oxidoestra-4-ene
• (E/Z)-3-(hydroxyimino)-6α,7α; 15α, 16α-bismethylene-17α-(1'-propenyl)-17β-3'-oxidoestra-4-ene
• (E/Z)-3-(hydroxyimino)-6β,7β; 15α, 16α-bismethylene-17α-(1'-propenyl)-17β-3'-oxidoestra-4-ene
• (E/Z)-3-(hydroxyimino)-6α,7α; 15β,16β-bismethylene-17α-(1'-propenyl)-17β-3'-oxidoestra-4-ene
• (E/Z)-3-(hydroxyimino)-6β,7β; 15β,16β-bismethylene-17α-(1'-propenyl)-17β-3'-oxidoestra-4-ene
• (E/Z)-3-(hydroxyimino)-17α-(1'-propenyl)-15α,16α-methylene-17β-3'-oxidoestra-4,6-diene
• (E/Z)-3-(hydroxyimino)-17α-(1'-propenyl)-15β,16β-methylene-17β-3'-oxidoestra-4,6-diene
• 17α-(1'-propenyl)-18-methyl-15α,16α-methylene-17β-3'-oxidoestra-4-en-3-one
• 17α-(1'-propenyl)-18-methyl-15β,16β-methylene-17β-3'-oxidoestra-4-en-3-one
• 7α,18-dimethyl-15α,16α-methylene-17α-(1'-propenyl)-17β-3'-oxidoestra-4-en-3-one
• 7β,18-dimethyl-15α,16α-methylene-17α-(1'-propenyl)-17β-3'-oxidoestra-4-en-3-one
• 7α,18-dimethyl-15β,16β-methylene-17α-(1'-propenyl)-17β-3'-oxidoestra-4-en-3-one
• 7β,18-dimethyl-15β,16β-methylene-17α-(1'-propenyl)-17α-3'-oxidoestra-4-en-3-one
• 7α-ethyl-18-methyl-15α,16α-methylene-17α-(1'-propenyl)-17β-3'-oxidoestra-4-en-3-one
• 7β-ethyl-18-methyl-15α,16α-methylene-17α-(1'-propenyl)-17β-3'-oxidoestra-4-en-3-one
• 7α-ethyl-18-methyl-15β,16β-methylene-17α-(1'-propenyl)-17β-3'-oxidoestra-4-en-3-one
• 7β-ethyl-18-methyl-15β,16β-methylene-17α-(1'-propenyl)-17β-3'-oxidoestra-4-en-3-one
• 7α-vinyl-18-methyl-15α,16α-methylene-17α-(1'-propenyl)-17β-3'-oxidoestra-4-en-3-one
• 7β-vinyl-18-methyl-15α,16α-methylene-17α-(1'-propenyl)-17β-3'-oxidoestra-4-en-3-one
• 7α-vinyl-18-methyl-15β,16β-methylene-17α-(1'-propenyl)-17β-3'-oxidoestra-4-en-3 -one
• 7β-vinyl-18-methyl-15β,16β-methylene-17α-(1'-propenyl)-17β-3'-oxidoestra-4-en-3-one
• 7α-cyclopropyl-18-methyl-15α,16α-methylene-17α-(1'-propenyl)-17β-3'-oxidoestra-4-en-3-one
• 7β-cyclopropyl-18-methyl-15α,16α-methylene-17α-(1'-propenyl)-17β-3'-oxidoestra-4-en-3-one
• 7α-cyclopropyl-18-methyl-15β,16β-methylene-17α-(1'-propenyl)-17β-3'-oxidoestra-4-en-3-one
• 7β-cyclopropyl-18-methyl-15β,16β-methylene-17α-(1'-propenyl)-17β-3'-oxidoestra-4-en-3-one
• 6-methylene-18-methyl-15α,16α-methylene-17α-(1'-propenyl)-17β-3'-oxido-estra-4-en-3-one
• 6-methylene-18-methyl-15β,16β-methylene-17α-(1'-propenyl)-17β-3'-oxido-estra-4-en-3-one
• 6,6-(1,2-ethanediyl)-18-methyl-15α,16α-methylene-17α-(1'-propenyl)-17β-3'-oxidoestra-4-en-3-one
• 6,6-(1,2-ethanediyl)-18-methyl-15β,16β-methylene-17α-(1'-propenyl)-17β-3'-oxidoestra-4-en-3-one
• 6α,7α; 15α,16α-bismethylene-18-methyl-17α-(1'-propenyl)-17β-3'-oxidoestra-4-en-3-one
• 6β,7β;15α,16a-bismethylene-18-methyl-17α-(1'-propenyl)-17β-3'-oxidoestra-4-en-3-one
• 6α,7α;15β,16β-bismethylene-18-methyl-17α-(1'-propenyl)-17β-3'-oxidoestra-4-en-3-one
• 6β,7β; 15β,16β-bismethylene-18-methyl-17α-(1'-propenyl)-17β-3'-oxidoestra-4-en-3-one
• 17α-(1'-propenyl)-18-methyl-15α,16a-methylene-17β-3'-oxidoestra-4,6-dien-3-one
• 17α-(1'-propenyl)-18-methyl-15β,16β-methylene-17β-3'-oxidoestra-4,6-dien-3-one
• (E/Z)-3-(hydroxyimino)-18-methyl-17α-(1'-propenyl)-15α,16α-methylene-17β-3'-oxidoestra-4-ene
• (E/Z)-3-(hydroxyimino)-18-methyl-17α-(1'-propenyl)-15β,16β-methylene-17β-3'-oxidoestra-4-ene
• (E/Z)-3-(hydroxyimino)-7α,18-bismethyl-15α,16α-methylene-17α-(1'-propenyl)-17β-3'-oxidoestra-4-ene
• (E/Z)-3-(hydroxyimino)-7β,18-bismethyl-15α,16α-methylene-17α-(1'-propenyl)-17β-3'-oxidoestra-4-ene
• (E/Z)-3-(hydroxyimino)-7α,18-bismethyl-15β,16β-methylene-17α-(1'-propenyl)-17β-3'-oxidoestra-4-ene
• (E/Z)-3-(hydroxyimino)-7β,18-bismethyl-15β,16β-methylene-17α-(1'-propenyl)-17β-3'-oxidoestra-4-ene
• (E/Z)-3-(hydroxyimino)-7α-ethyl-18-methyl-15α,16α-methylene-17α-(1'-propenyl)-17β-3'-oxidoestra-4-ene
• (E/Z)-3-(hydroxyimino)-7β-ethyl-18-methyl-15α,16α-methylene-17α-(1'-propenyl)-17β-3'-oxidoestra-4-ene
• (E/Z)-3-(hydroxyimino)-7α-ethyl-18-methyl-15β,16β-methylene-17α-(1'-propenyl)-17β-3'-oxidoestra-4-ene
• (E/Z)-3-(hydroxyimino)-7β-ethyl-18-methyl-15β,16β-methylene-17α-(1'-propenyl)-17β-3'-oxidoestra-4-ene
• (E/Z)-3-(hydroxyimino)-7α-vinyl-18-methyl-15α,16α-methylene-17α-(1'-propenyl)-17β-3'-oxidoestra-4-ene
• (E/Z)-3-(hydroxyimino)-7β-vinyl-18-methyl15α,16a-methylene-17α-(1'-propenyl)-17β-3'-oxidoestra-4-ene
• (E/Z)-3-(hydroxyimino)-7α-vinyl-18-methyl-15β,16β-methylene-17α-(1'-propenyl)-17β-3'-oxidoestra-4-ene
• (E/Z)-3-(hydroxyimino)-7β-vinyl-18-methyl-15β,16β-methylene-17α-(1'-propenyl)-17β-3'-oxidoestra-4-ene
• (E/Z)-3-(hydroxyimino)-7α-cyclopropyl-18-methyl-15α,16α-methylene-17α-(1'-propenyl)-17β-3'-oxidoestra-4-ene
• (E/Z)-3-(hydroxyimino)-7β-cyclopropyl-18-methyl-15α,16α-methylene-17α-(1'-propenyl)-17β-3'-oxidoestra-4-ene
• (E/Z)-3-(hydroxyimino)-7α-cyclopropyl-18-methyl-15β,16β-methylene-17α-(1'-propenyl)-17β-3'-oxidoestra-4-ene
• (E/Z)-3-(hydroxyimino)-7β-cyclopropyl-18-methyl-15β,16β-methylene-17α-(1'-propenyl)-17β-3'-oxidoestra-4-ene
• (E/Z)-3-(hydroxyimino)-18-methyl-6-methylene-17α-(1'-propenyl)-15α,16α-methylene-17β-3'-oxidoestra-4-ene
• (E/Z)-3-(hydroxyimino)-18-methyl-6-methylene-17α-(1'-propenyl)-15β,16β-methylene-17β-3'-oxidoestra-4-ene
• (E/Z)-3-(hydroxyimino)-6,6-(1,2-ethandiyl)-17α-(1'-propenyl)-18-methyl-15α,16α-methylene-17β-3'-oxidoestra-4-ene
• (E/Z)-3-(hydroxyimino)-6,6-(1,2-ethandiyl)-17α-(1'-propenyl)-18-methyl-15β,16β-methylene-17β**-**3'-oxidoestra-4-ene
• (E/Z)-3-(hydroxyimino)-6α,7α;15α,16α-bismethylene-18-methyl-17α-(1'-propenyl)-17β-3'-oxidoestra-4-ene
• (E/Z)-3-(hydroxyimino)-6β,7β; 15α,16α-bismethylene-18-methyl-17α-(1'-propenyl)-17β-3'-oxidoestra-4-ene
• (E/Z)-3-(hydroxyimino)-6α,7α; 15β, 16β-bismethylene-18-methyl-17α-(1'-propenyl)-17β-3'-oxidoestra-4-ene
• (E/Z)-3-(hydroxyimino)-6β,7β; 15β,16β-bismethylene-18-methyl-17α-(1'-propenyl)-17β-3'-oxidoestra-4-ene
• (E/Z)-3-(hydroxyimino)-17α-(1'-propenyl)-18-methyl-15α, 16α-methylene-17β-3'-oxidoestra-4,6-diene
• (E/Z)-3-(hydroxyimino)-17α-(1'-propenyl)-18-methyl-15β,16β-methylene-17β-3'-oxidoestra-4,6-diene

11. 15,16-Methylene-17-(1'-propenyl)-17-3'-oxidoestra-4-en-3-one derivative according to one of the previous claims for oral contraception and for the treatment of pre-, peri- and postmenopausal problems.

12. Use of the 15,16-methylene-17-(1'-propenyl)-17-3'-oxidoestra-4-en-3-one derivative according to one of Claims 1 - 11 for the production of a drug for oral contraception and for the treatment of pre-, peri- and postmenopausal problems.

13. Use according to Claim 12, **characterized in that** the drug has progestational, antimineralcorticoid and neutral to slight androgenic activity.

14. Drug containing at least one 15,16-methylene-17-(1'-propenyl)-17-3'-oxidoestra-4-en-3-one derivative according to one of Claims 1 - 11 and at least one suitable pharmaceutically harmless additive.

15. Drug according to Claim 14, also containing at least one estrogen.

16. Drug according to Claim 15, **characterized in that** the estrogen is ethynylestradiol.

17. Drug according to Claim 15, **characterized in that** the estrogen is estradiol valerate.

18. Drug according to Claim 15, **characterized in that** the estrogen is a natural estrogen.

19. Drug according to Claim 18, **characterized in that** the natural estrogen is estradiol.

20. Drug according to Claim 18, **characterized in that** the natural estrogen is a conjugated estrogen.

21. Use of the 15,16-methylene-17-(1'-propenyl)-17-3'-oxidoestra-4-en-3-one derivative according to any of Claims 1-9 for the production of a medicinal product for intrauterine use.

22. Use according to Claim 21 for the production of an intrauterine system (IUS).

23. Drug containing at least one 15,16-methylene-17-(1'-propenyl)-17-3'-oxidoestra-4-en-3-one derivative according to one of Claims 1 - 9 and at least one suitable pharmaceutically harmless additive, **characterized in that** it is designed for intrauterine use.

24. Drug according to Claim 23, **characterized in that** it is an intrauterine system.

## Revendications

1. Dérivé de 15,16-méthylène-17-(1'-propényl)-17-3'-oxydoestra-4-én-3-one de formule chimique générale I 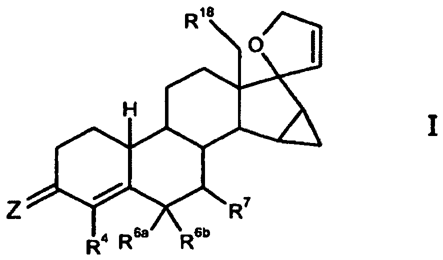 dans laquelle
Z est choisi dans le groupe comprenant oxygène, deux atomes d'hydrogène, NOR' ou NNHSO₂R', R' étant hydrogène, alkyle en C₁-C₁₀, aryle et aralkyle en C₇-C₂₀, R⁴ est choisi dans le groupe comprenant hydrogène, fluor, chlore ou brome,
et soit :
R^{6a}, R^{6b} sont choisis à chaque fois indépendamment l'un de l'autre dans le groupe comprenant hydrogène, alkyle en C₁-C₁₀, alcényle en C₂-C₁₀ et alcynyle en C₂-C₁₀, ou forment ensemble un méthylène ou un 1,2-éthanediyle, et
R⁷ est choisi dans le groupe comprenant hydrogène, alkyle en C₁-C₁₀, cycloalkyle en C₃-C₆, alcényle en C₂-C₁₀ et alcynyle en C₂-C₁₀,
soit :
R^{6a}, R⁷ forment ensemble un oxygène ou un groupe méthylène, ou sont omis avec formation d'une double liaison entre C⁶ et C⁷ et
R^{6b} est choisi dans le groupe comprenant hydrogène, alkyle en C₁-C₁₀, alcényle en C₂-C₁₀ et alcynyle en C₂-C₁₀, et
R¹⁸ est choisi dans le groupe comprenant hydrogène et alkyle en C₁-C₃,
ainsi que leurs solvates, hydrates et sels, et y compris toutes les formes cristallines et tous les stéréoisomères.

2. Dérivé de 15,16-méthylène-17-(1'-propényl)-17-3'-oxydoestra-4-én-3-one selon la revendication 1, **caractérisé en ce que** Z est choisi dans le groupe comprenant oxygène, NOR' et NNHSO₂R'.

3. Dérivé de 15,16-méthylène-17-(1'-propényl)-17-3'-oxydoestra-4-én-3-one selon l'une quelconque des revendications précédentes, **caractérisé en ce que** Z représente l'oxygène.

4. Dérivé de 15,16-méthylène-17-(1'-propényl)-17-3'-oxydoestra-4-én-3-one selon l'une quelconque des revendications précédentes, **caractérisé en ce que** R⁴ est choisi dans le groupe comprenant l'hydrogène et le chlore.

5. Dérivé de 15,16-méthylène-17-(1'-propényl)-17-3'-oxydoestra-4-én-3-one selon l'une quelconque des revendications précédentes, **caractérisé en ce que** R^{6a} et R^{6b} forment ensemble un 1,2-éthanediyle ou sont chacun l'hydrogène.

6. Dérivé de 15,16-méthylène-17-(1'-propényl)-17-3'-oxydoestra-4-én-3-one selon l'une quelconque des revendications précédentes, **caractérisé en ce que** R⁷ est choisi dans le groupe comprenant hydrogène, méthyle, éthyle et vinyle.

7. Dérivé de 15,16-méthylène-17-(1'-propényl)-17-3'-oxydoestra-4-én-3-one selon l'une quelconque des revendications 1 à 4, **caractérisé en ce que** R^{6a} et R⁷ forment ensemble un groupe méthylène.

8. Dérivé de 15,16-méthylène-17-(1'-propényl)-17-3'-oxydoestra-4-én-3-one selon l'une quelconque des revendications 1 à 4, **caractérisé en ce que** R^{6a} et R⁷ sont omis avec formation d'une double liaison entre C⁶ et C⁷.

9. Dérivé de 15,16-méthylène-17-(1'-propényl)-17-3'-oxydoestra-4-én-3-one selon l'une quelconque des revendications précédentes, **caractérisé en ce que** R¹⁸ est choisi dans le groupe comprenant hydrogène et méthyle.

10. Dérivé de 15,16-méthylène-17-(1'-propényl)-17-3'-oxydoestra-4-én-3-one selon l'une quelconque des revendications précédentes, choisi dans le groupe comprenant :
• la 17α-(1'-propényl)-15α,16α-méthylène-17β-3'-oxydoestra-4-én-3-one
• la 17α-(1'-propényl)-15β,16β-méthylène-17β-3'-oxydoestra-4-én-3-one
• la 7α-méthyl-15α,16α-méthylène-17α-(1'-propényl)-17β-3'-oxydoestra-4-én-3-one
• la 7β-méthyl-15α,16α-méthylène-17α-(1'-propényl)-17β-3'-oxydoestra-4-én-3-one
• la 7α-méthyl-15β,16β-méthylène-17α-(1'-propényl)-17β-3'-oxydoestra-4-én-3-one
• la 7β-méthyl-15β,16β-méthylène-17α-(1'-propényl)-17β-3'-oxydoestra-4-én-3-one
• la 7α-éthyl-15α,16α-méthylène-17α-(1'-propényl)-17β-3'-oxydoestra-4-én-3-one
• la 7β-éthyl-15α,16α-méthylène-17α-(1'-propényl)-17β-3'-oxydoestra-4-én-3-one
• la 7α-éthyl-15β,16β-méthylène-17α-(1'-propényl)-17β-3'-oxydoestra-4-én-3-one
• la 7β-éthyl-15β,16β**-**méthylène-17α-(1'-propényl)-17β-3'-oxydoestra-4-én-3-one
• la 7α-vinyl-15α,16α-méthylène-17α-(1'-propényl)-17β-3'-oxydoestra-4-én-3-one
• la 7β-vinyl-15α,16α-méthylène-17α-(1'-propényl)-17β-3'-oxydoestra-4-én-3-one
• la 7α-vinyl-15β,16β-méthylène-17α-(1'-propényl)-17β-3'-oxydoestra-4-én-3-one
• la 7β-vinyl-15β,16β-méthylène-17a-(1'-propényl)-17α-3'-oxydoestra-4-én-3-one
• la 7α-cyclopropyl-15α,16α-méthylène-17α-(1'-propényl)-17β-3'-oxydoestra-4-én-3-one
• la 7β-cyclopropyl-15α,16α-méthylène-17α-(1'-propényl)-17β-3'-oxydoestra-4-én-3-one
• la 7α-cyclopropyl-15β,16β-méthylène-17α-(1'-propényl)-17β-3'-oxydoestra-4-én-3-one
• la 7β-cyclopropyl-15β,16β-méthylène-17α-(1'-propényl)-17β-3'-oxydoestra-4-én-3-one
• la 6-méthylène-15α,16α-méthylène-17α-(1'-propényl)-17β-3'-oxydoestra-4-én-3-one
• la 6-méthylène-15β,16β-méthylène-17α-(1'-propényl)-17β-3'-oxydoestra-4-én-3-one
• la 6,6-(1,2-éthanediyl)-15α,16α-méthylène-17α-(1'-propényl)-17β-3'-oxydoestra-4-én-3-one
• la 6,6-(1,2-éthanediyl)-15β,16β-méthylène-17α-(1'-propényl)-17β-3'-oxydoestra-4-én-3-one
• la 6α,7α;15α,16α-bisméthylène-17α-(1'-propényl)-17β-3'-oxydoestra-4-én-3-one
• la 6β,7β;15α,16α-bisméthylène-17α-(1'-propényl)-17β-3'-oxydoestra-4-én-3-one
• la 6α,7α;15β,16β-bisméthylène-17α-(1'-propényl)-17β-3'-oxydoestra-4-én-3-one
• la 6β,7β;15β,16β-bisméthylène-17α-(1'-propényl)-17β-3'-oxydoestra-4-én-3-one
• la 17α-(1'-propényl)-15α,16α-méthylène-17β-3'-oxydoestra-4,6-dién-3-one
• la 17α-(1'-propényl)-15β,16β-méthylène-17β-3'-oxydoestra-4,6-dién-3-one
• le (E/Z)-3-(hydroxyimino)-17α-(1'-propényl)-15α,16α-méthylène-17β-3'-oxydoestra-4-ène
• le (E/Z)-3-(hydroxyimino)-17α-(1'-propényl)-15β,16β-méthylène-17β-3'-oxydoestra-4-ène
• le (E/Z)-3-(hydroxyimino)-7α-méthyl-15α,16α-méthylène-17α-(1'-propényl)-17β-3'-oxydoestra-4-ène
• le (E/Z)-3-(hydroxyimino)-7β-méthyl-15α,16α-méthylène-17α-(1'-propényl)-17β-3'-oxydoestra-4-ène
• le (E/Z)-3-(hydroxyimino)-7α-méthyl-15β,16β-méthylène-17α-(1'-propényl)-17β-3'-oxydoestra-4-ène
• le (E/Z)-3-(hydroxyimino)-7β-méthyl-15β,16β-méthylène-17α-(1'-propényl)-17β-3'-oxydoestra-4-ène
• le (E/Z)-3-(hydroxyimino)-7α-éthyl-15α,16α-méthylène-17α-(1'-propényl)-17β-3'-oxydoestra-4-ène
• le (E/Z)-3-(hydroxyimino)-7β-éthyl-15α,16α-méthylène-17α-(1'-propényl)-17β-3'-oxydoestra-4-ène
• le (E/Z)-3-(hydroxyimino)-7α-éthyl-15β,16β-méthylène-17α-(1'-propényl)-17β-3'-oxydoestra-4-ène
• le (E/Z)-3-(hydroxyimino)-7β-éthyl-15β,16β-méthylène-17α-(1'-propényl)-17β-3'-oxydoestra-4-ène
• le (E/Z)-3-(hydroxyimino)-7α-vinyl-15α,16α-méthylène-17α-(1'-propényl)-17β-3'-oxydoestra-4-ène
• le (E/Z)-3-(hydroxyimino)-7β-vinyl-15α,16α-méthylène-17α-(1'-propényl)-17β-3'-oxydoestra-4-ène
• le (E/Z)-3-(hydroxyimino)-7α-vinyl-15β,16β-méthylène-17α-(1'-propényl)-17β-3'-oxydoestra-4-ène
• le (E/Z)-3-(hydroxyimino)-7β-vinyl-15β,16β-méthylène-17α-(1'-propényl)-17β-3'-oxydoestra-4-ène
• le (E/Z)-3-(hydroxyimino)-7α-cyclopropyl-15α,16α-méthylène-17α-(1'-propényl)-17β-3'-oxydoestra-4-ène
• le (E/Z)-3-(hydroxyimino)-7β-cyclopropyl-15α,16α-méthylène-17α-(1'-propényl)-17β-3'-oxydoestra-4-ène
• le (E/Z)-3-(hydroxyimino)-7α-cyclopropyl-15β,16β-méthylène-17α-(1'-propényl)-17β-3'-oxydoestra-4-ène
• le (E/Z)-3-(hydroxyimino)-7β-cyclopropyl-15β,16β-méthylène-17α-(1'-propényl)-17β-3'-oxydoestra-4-ène
• le (E/Z)-3-(hydroxyimino)-6-méthylène-17α-(1'-propényl)-15α,16α-méthylène-17β-3'-oxydoestra-4-ène
• le (E/Z)-3-(hydroxyimino)-6-méthylène-17α-(1'-propényl)-15β,16β-méthylène-17β-3'-oxydoestra-4-ène
• le (E/Z)-3-(hydroxyimino)-6,6-(1,2-éthanediyl)-17β-(1'-propényl)-15α,16α-méthylène-17β-3'-oxydoestra-4-ène
• le (E/Z)-3-(hydroxyimino)-6,6-(1,2-éthanediyl)-17α-(1'-propényl)-15β,16β-méthylène-17β-3'-oxydoestra-4-ène
• le (E/Z)-3-(hydroxyimino)-6α,7α;15α,16α-bisméthylène-17α-(1'-propényl)-17β-3'-oxydoestra-4-ène
• le (E/Z)-3-(hydroxyimino)-6β,7β; 15α,16α-bisméthylène-17α-(1'-propényl)-17β-3'-oxydoestra-4-ène
• le (E/Z)-3-(hydroxyimino)-6α,7α;15β,16β-bisméthylène-17α-(1'-propényl)-17β-3'-oxydoestra-4-ène
• le (E/Z)-3-(hydroxyimino)-6β,7β;15β,16β-bisméthylène-17α-(1'-propényl)-17β-3'-oxydoestra-4-ène
• le (E/Z)-3-(hydroxyimino)-17α-(1'-propényl)-15α,16α-méthylène-17β-3'-oxydoestra-4,6-diène
• le (E/Z)-3-(hydroxyimino)-17α-(1'-propényl)-15β,16β-méthylène-17β-3'-oxydoestra-4,6-diène
• la 17α-(1'-propényl)-18-méthyl-15α,16α-méthylène-17β-3'-oxydoestra-4-én-3-one
• la 17α-(1'-propényl)-18-méthyl-15β,16β-méthylène-17β-3'-oxydoestra-4-én-3-one
• la 7α,18-diméthyl-15α,16α-méthylène-17a-(1'-propényl)-17β-3'-oxydoestra-4-én-3-one
• la 7β,18-diméthyl-15α,16α-méthylène-17α-(1'-propényl)-17β-3'-oxydoestra-4-én-3-one
• la 7α,18-diméthyl-15β,16β-méthylène-17α-(1'-propényl)-17β-3'-oxydoestra-4-én-3-one
• la 7β,18-diméthyl-15β,16β-méthylène-17α-(1'-propényl)-17β-3'-oxydoestra-4-én-3-one
• la 7α-éthyl-18-méthyl-15α,16α-méthylène-17α-(1'-propényl)-17β-3'-oxydoestra-4-én-3-one
• la 7β-éthyl-18-méthyl-15α,16α-méthylène-17α-(1'-propényl)-17β-3'-oxydoestra-4-én-3-one
• la 7α-éthyl-18-méthyl-15β,16β-méthylène-17α-(1'-propényl)-17β-3'-oxydoestra-4-én-3-one
• la 7β-éthyl-18-méthyl-15β,16β-méthylène-17a-(1'-propényl)-17β-3'-oxydoestra-4-én-3-one
• la 7α-vinyl-18-méthyl-15α,16α-méthylène-17α-(1'-propényl)-17β-3'-oxydoestra-4-én-3-one
• la 7β-vinyl-18-méthyl-15α,16α-méthylène-17α-(1'-propényl)-17β-3'-oxydoestra-4-én-3-one
• la 7α-vinyl-18-méthyl-15β,16β-méthylène-17α-(1'-propényl)-17β-3'-oxydoestra-4-én-3-one
• la 7β-vinyl-18-méthyl-15β,16β-méthylène-17α-(1'-propényl)-17β-3'-oxydoestra-4-én-3-one
• la 7α-cyclopropyl-18-méthyl-15α,16α-méthylène-17α-(1'-propényl)-17β-3'-oxydoestra-4-én-3-one
• la 7β-cyclopropyl-18-méthyl-15α,16β-méthylène-17α-(1'-propényl)-17β-3'-oxydoestra-4-én-3-one
• la 7α-cyclopropyl-18-méthyl-15β,16β-méthylène-17a-(4'-propényl)-17β-3'-oxydoestra-4-én-3-one
• la 7β-cyclopropyl-18-méthyl-15β,16β-méthylène-17α-(1'-propényl)-17β-3'-oxydoestra-4-én-3-one
• la 6-méthylène-18-méthyl-15α,16a-méthylène-17α-(1'-propényl)-17β-3'-oxydoestra-4-én-3-one
• la 6-méthylène-18-méthyl-15β,16β-méthylène-17α-(1'-propényl)-17β-3'-oxydoestra-4-én-3-one
• la 6,6-(1,2-éthanediyl)-18-méthyl-15α,16α-méthylène-17α-(1'-propényl)-17β-3'-oxydoestra-4-én-3-one
• la 6,6-(1,2-éthanediyl)-18-méthyl-15β,16β-méthylène-17α-(1'-propényl)-17β-3'-oxydoestra-4-én-3-one
• la 6α,7α;15α,16α-bisméthylène-18-méthyl-17α-(1'-propényl)-17β-3'-oxydoestra-4-én-3-one
• la 6β,7β;15α,16α-bisméthylène-18-méthyl-17α-(1'-propényl)-17β-3'-oxydoestra-4-én-3-one
• la 6α,7α;15β,16β-bisméthylène-18-méthyl-17α-(1'-propényl)-17β-3'-oxydoestra-4-én-3-one
• la 6β,7β;15β,16β-bisméthylène-18-méthyl-17α-(1'-propényl)-17β-3'-oxydoestra-4-én-3-one
• la 17α-(1'-propényl)-18-méthyl-15α,16α-méthylène-17β-3'-oxydoestra-4,6-dién-3-one
• la 17α-(1'-propényl)-18-méthyl-15α,16α-méthylène-17α-3'-oxydoestra-4,6-dién-3-one
• le (E/Z)-3-(hydroxyimino)-18-méthyl-17α-(1'-propényl)-15α,16α-méthylène-17β-3'-oxydoestra-4-ène
• le (E/Z)-3-(hydroxyimino)-18-méthyl-17α-(1'-propényl)-15β,16β-méthylène-17β-3'-oxydoestra-4-ène
• le (E/Z)-3-(hydroxyimino)-7α,18-bisméthyl-15α,16α-méthylène-17α-(1'-propényl)-17β-3'-oxydoestra-4-ène
• le (E/Z)-3-(hydroxyimino)-7β,18-bisméthyl-15α,16α-méthylène-17α-(1'-propényl)-17β-3'-oxydoestra-4-ène
• le (E/Z)-3-(hydroxyimino)-7α,18-bisméthyl-15β,16β-méthylène-17a-(1'-propényl)-17β-3'-oxydoestra-4-ène
• le (E/Z)-3-(hydroxyimino)-7β,18-bisméthyl-15β,16β-méthylène-17α-(1'-propényl)-17β-3'-oxydoestra-4-ène
• le (E/Z)-3-(hydroxyimino)-7α-éthyl-18-méthyl-15α,16α-méthylène-17α-(1'-propényl)-17β-3'-oxydoestra-4-ène
• le (E/Z)-3-(hydroxyimino)-7α-éthyl-18-méthyl-15α,16α-méthylène-17α-(1'-propényl)-17β-3'-oxydoestra-4-ène
• le (E/Z)-3-(hydroxyimino)-7α-éthyl-18-méthyl-15β,16β-méthylène-17α-(1'-propényl)-17β-3'-oxydoestra-4-ène
• le (E/Z)-3-(hydroxyimino)-7β-éthyl-18-méthyl-15β,16β-méthylène-17α-(1'-propényl)-17β-3'-oxydoestra-4-ène
• le (E/Z)-3-(hydroxyimino)-7α-vinyl-18-méthyl-15α,16α-méthylène-17α-(1'-propényl)-17β-3'-oxydoestra-4-ène
• le (E/Z)-3-(hydroxyimino)-7β-vinyl-18-méthyl-15α,16α-méthylène-17α-(1'-propényl)-17β-3'-oxydoestra-4-ène
• le (E/Z)-3-(hydroxyimino)-7a-vinyl-18-méthyl-15β,16β-méthylène-17α-(1'-propényl)-17β-3'-oxydoestra-4-ène
• le (E/Z)-3-(hydroxyimino)-7β-vinyl-18-méthyl-15β,16β-méthylène-17α-(1'-propényl)-17β-3'-oxydoestra-4-ène
• le (E/Z)-3-(hydroxyimino)-7α-cyclopropyl-18-méthyl-15α,16α-méthylène-17α-(1'-propényl)-17β-3'-oxydoestra-4-ène
• le (E/Z)-3-(hydroxyimino)-7β-cyclopropyl-18-méthyl-15α,16α-méthylène-17α-(1'-propényl)-17β-3'-oxydoestra-4-ène
• le (E/Z)-3-(hydroxyimino)-7α-cyclopropyl-18-méthyl-15β,16β-méthylène-17α-(1'-propényl)-17β-3'-oxydoestra-4-ène
• le (E/Z)-3-(hydroxyimino)-7β-cyclopropyl-18-méthyl-15β,16β-méthylène-17α-(1'-propényl)-17β-3'-oxydoestra-4-ène
• le (E/Z)-3-(hydroxyimino)-18-méthyl-6-méthylène-17α-(1'-propényl)-15α,16α-méthylène-17β-3'-oxydoestra-4-ène
• le (E/Z)-3-(hydroxyimino)-18-méthyl-6-méthylène-17α-(1'-propényl)-15β,16β-méthylène-17β-3'-oxydoestra-4-ène
• le (E/Z)-3-(hydroxyimino)-6,6-(1,2-éthanediyl)-17α-(1'-propényl)-18-méthyl-15α,16α-méthylène-17β-3'-oxydoestra-4-ène
• le (E/Z)-3-(hydroxyimino)-6,6-(1,2-éthanediyl)-17α-(1'-propényl)-18-méthyl-15β,16β-méthylène-17β-3'-oxydoestra-4-ène
• le (E/Z)-3-(hydroxyimino)-6α,7α;15α,16α-bisméthylène-18-méthyl-17a-(1'-propényl)-17β-3'-oxydoestra-4-ène
• le (E/Z)-3-(hydroxyimino)-6β,7β;15α,16α-bisméthylène-18-méthyl-17a-(1'-propényl)-17β-3'-oxydoestra-4-ène
• le (E/Z)-3-(hydroxyimino)-6α,7α;15β,16β-bisméthylène-18-méthyl-17α-(1'-propényl)-17β-3'-oxydoestra-4-ène
• le (E/Z)-3-(hydroxyimino)-6β,7β;15β,16β-bisméthylène-18-méthyl-17α-(1'-propényl)-17β-3'-oxydoestra-4-ène
• le (E/Z)-3-(hydroxyimino)-17α-(1'-propényl)-18-méthyl-15α,16α-méthylène-17β-3'-oxydoestra-4,6-diène
• le (E/Z)-3-(hydroxyimino)-17α-(1'-propényl)-18-méthyl-15β,16β-méthylène-17β-3'-oxydoestra-4,6-diène.

11. Dérivé de 15,16-méthylène-17-(1'-propényl)-17-3'-oxydoestra-4-én-3-one selon l'une quelconque des revendications précédentes pour la contraception orale et pour le traitement des troubles de la pré-, péri- et post-ménopause.

12. Utilisation du dérivé de 15,16-méthylène-17-(1'-propényl)-17-3'-oxydoestra-4-én-3-one selon l'une quelconque des revendications 1 à 11 pour la fabrication d'un médicament pour la contraception orale et pour le traitement des troubles de la pré-, péri- et post-ménopause.

13. Utilisation selon la revendication 12, **caractérisée en ce que** le médicament présente une action gestagène, antiminéralocorticoïde et androgène neutre à légère.

14. Médicament, contenant au moins un dérivé de 15,16-méthylène-17-(1'-propényl)-17-3'-oxydoestra-4-én-3-one selon l'une quelconque des revendications 1 à 11 et au moins un additif pharmaceutiquement inoffensif approprié.

15. Médicament selon la revendication 14, contenant également au moins un estrogène.

16. Médicament selon la revendication 15, **caractérisé en ce que** l'estrogène est l'éthinylestradiol.

17. Médicament selon la revendication 15, **caractérisé en ce que** l'estrogène est le valérate d'estradiol.

18. Médicament selon la revendication 15, **caractérisé en ce que** l'estrogène est un estrogène naturel.

19. Médicament selon la revendication 18, **caractérisé en ce que** l'estrogène naturel est l'estradiol.

20. Médicament selon la revendication 18, **caractérisé en ce que** l'estrogène naturel est un estrogène conjugué.

21. Utilisation du dérivé de 15,16-méthylène-17-(1'-propényl)-17-3'-oxydoestra-4-én-3-one selon l'une quelconque des revendications 1 à 9 pour la fabrication d'un médicament pour utilisation intra-utérine.

22. Utilisation selon la revendication 21 pour la fabrication d'un système intra-utérin (SIU).

23. Médicament contenant au moins un dérivé de 15,16-méthylène-17-(1'-propényl)-17-3'-oxydoestra-4-én-3-one selon l'une quelconque des revendications 1 à 9 et au moins un véhicule pharmaceutiquement inoffensif approprié, **caractérisé en ce qu'**il est préparé pour l'utilisation intra-utérine.

24. Médicament selon la revendication 23, **caractérisé en ce qu'**il s'agit d'un système intra-utérin.
